# EUROPEAN PATENT APPLICATION

(11) **EP 2 082 754 A1**
(43) Date of publication of application: **29.07.2009**
(21) Application number: 07828003.9
(22) Date of filing: 08.11.2007
(51) Int. Cl.: A61L 27/00, A61K 9/00, A61K 45/00, A61P 19/00, A61P 19/02, A61P 31/04, A61P 35/00

(54) **BONE DEFECT FILLER, RELEASE-CONTROLLED CARRIER, AND THEIR PRODUCTION METHODS**

(30) Priority: 11.11.2006 JP 2006306070
(71) Applicant: The University of Tokyo, Bunkyo-ku, Tokyo 1138654 (JP); Next21 K.K., Tokyo 113-0033 (JP)
(72) Inventor: SASAKI, Nobuo, Tokyo 113-8654 (JP); TEI, Yuichi, Tokyo 113-8654 (JP); IGAWA, Kazuyo, Tokyo 113-8654 (JP); SUZUKI, Shigeki, 3-38-1, Hongo, Bunkyo-ku Tokyo 113-0033 (JP); SHIMIZU, Koutaro, 3-38-1, Hongo, Bunkyo-ku Tokyo 113-0033 (JP)
(74) Representative: Pitchford, James Edward
(86) International application number: PCT/JP2007/001225
(87) International publication number: WO 2008/065738

(57) **Abstract**

A bone filling material having controlled release of a pharmaceutical agent such as a growth factor, and a drug release controlling carrier having controlled release of a pharmaceutical agent is provided by inactivating a functional group which can strongly bind to the pharmaceutical agent of the bone filling material with a blocking agent. The bone filling material and the drug release controlling carrier of the present invention comprises: a calcium-based material such as hydroxyapatite, carbonate apatite, fluorapatite, chlorapatite, β-TCP, and α-TCP; a blocking agent of the calcium-based material such as serine and dextran; and a pharmaceutical agent.

## Description

### BACKGROUND OF THE INVENTION

### Field of the Invention

The present invention relates to a bone filling material, a release controlling carrier, and methods for producing thereof. In particular, the present invention relates to a bone filling material and a release controlling carrier whose drug release are controlled by blocking agents, and methods for producing thereof.

### Description of the Related Art

In case one suffered a partial bone defect in an accident or in a surgical treatment, reproduction of the bone defect site has been promoted, for example, by filling bone filling materials which are turned into bone tissues in the defect site. In a medical treatment of bone deformity, bone reproduction has also been promoted by filling bone filling materials in the deformed site. As a raw material of the bone filling material, calcium phosphate-based materials such as hydroxyapatite and β-TCP, or biodegradable plastic such as polylactic acid have been used.

Japanese Unexamined Patent Application Publication No. 8-224261 discloses a method for reproducing, increasing, and replacing bones, and a structural member used for the method. What is called a tetrapod^{™}-shaped bone filling material having pluralities of protruding parts is disclosed in this bulletin.

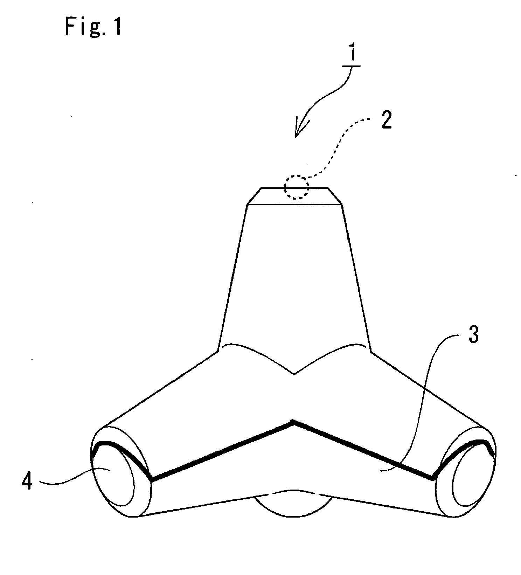

Japanese Unexamined Patent Application Publication No. 2004-97259 discloses a tetrapod^{™}-shaped bone filling material (bone fixing material) (e.g., fig.1 to fig.3 of the bulletin).

Japanese Unexamined Patent Application Publication (Translation of PCT Application) No. 2004-524090 discloses a method for producing a transplant bone substitute containing a growth factor, an antibiotic, or the like. The growth factor or the like, however, is difficult to be released because it is absorbed by the transplant bone substitute.

Calcium phosphate cements functioning as DDS such as a pharmaceutical agent and an antibody is disclosed in M.P. Ginebra, T. Traykova, and J.A. Planell "Calcium phosphate cements as bone drug delivery systems: A review", J. Controlled Release 113 (2006) 102-110.

On the other hand, biologically active substance such as a growth factor ordinary shows activity only when the concentration reaches some level, and then it keeps showing the activity in conditions where the certain level of the concentration is maintained. Therefore, a preferred bone filling material having the biological active substance is that releases the biological active substance by a predetermined concentration thereof in a short period after the material is filled in a bone defect site, and then maintains the release at a certain concentration.

Therefore, the object of the present invention is to provide a bone filling material whose drug release (e.g., release of a growth factor) is controlled.

The object of the present invention is also to provide a release controlling carrier whose drug release is controlled.

### SUMMARY OF THE INVENTION

The present invention is basically based on the following idea. The absorbability between a pharmaceutical agent and a bone filling material can be controlled by inactivating a functional group which can strongly bind to a pharmaceutical agent existing in a bone filling material by a blocking agent, and therefore, release of a growth factor and the like can be controlled. In particular, the present invention relates to a bone filling material comprising a calcium-based material, a blocking agent of the calcium-based material, and a pharmaceutical agent; a method for producing the bone filling material; a drug release controlling carrier comprising calcium-based material and a blocking agent of the calcium-based material.

In the present invention, a bone filling material whose drug release (e.g., growth factor release) is controlled, and a method for producing thereof can be provided.

In the present invention, a release controlling carrier whose drug release is controlled can also be provided.

### BREIF DESCRIPTION OF THE DRAWINGS

Fig. 1 is a conceptual diagram showing an example of a mold used in the method for producing a bone filling material of the present invention.
Fig. 2 is a conceptual diagram for explaining an example of a tetrapod-shaped bone filling material. Fig. 2(A) is a side view, Fig. 2(B) is a top view, and Fig. 2(C) is a perspective view.
Fig. 3 is a conceptual diagram for explaining an example of a tetrapod-shaped bone filling material with its top portion cut off. Fig. 3(A) and Fig. 3(B) are side views,
Fig. 3(C) is a top view, and Fig. 3(D) is a perspective view.
Fig. 4 is a graph showing results of a bending strength test of test pieces.
Fig. 5 is a graph showing absorbance to evaluate drug release in an in vitro experiment.
Fig. 6 is a photograph, in place of a drawing, showing a mouse skull whereon circular all layer bone defect part is formed.

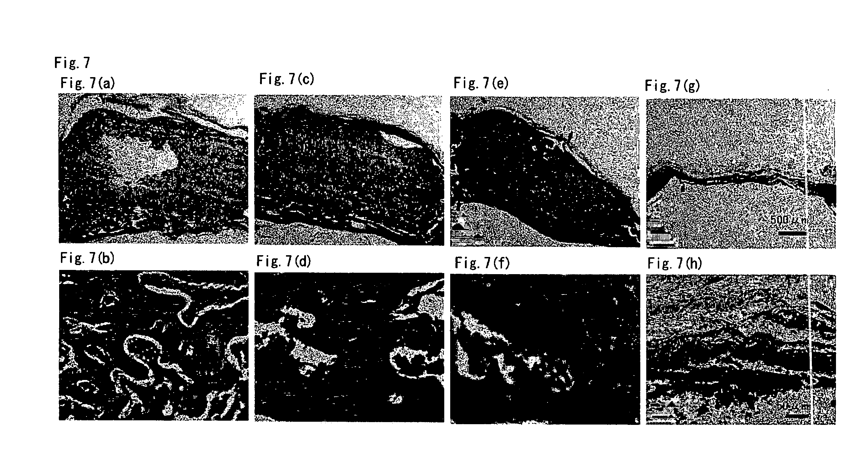

Fig. 7 is an X-ray photograph, in place of a drawing, showing a cross-sectional surface of a skull. Fig. 7(a) shows a skull whose circular all layer bone defect part is filled with a bone filling material containing 5% of serine as a blocking agent. Fig. 7(b) is a partial enlarged view of Fig. 7(a). Fig. 7(c) shows a skull whose circular all layer bone defect part is filled with a bone filling material containing 5% of trehalose as a blocking agent. Fig. 7(d) is a partial enlarged view of Fig. 7(c). Fig. 7(e) shows a skull whose circular all layer bone defect part is filled with a bone filling material containing 1% of dextran as a blocking agent. Fig. 7(f) is a partial enlarged view of Fig. 7(e). Fig. 7(g) shows a skull whose circular all layer bone defect part is filled with a bone filling material without containing a blocking agent. Fig. 7(h) is a partial enlarged view of Fig. 7(g).
Fig. 8 is a graph, in place of a diagram, showing a result of in vitro quantification test showing release control of BMP.

### Reference Numeral

1. shape of the mold with its grooves
2. part corresponding to an injection hole
3. parting face
4. wedge
11. bone filling material
12. protruding part
13. tip part

### DESCRIPTION OF THE PREFERRED EMBODIMENTS

### Bone Filling Material

The first aspect of the present invention relates to a bone filling material comprising: a calcium-based material; a blocking agent of the calcium-based material; and a pharmaceutical agent. The bone filling material is to be injected in a bone defect site or a bone deformation part, or is to be embedded as an implant. It has a characteristics of being replaced with bone tissues gradually. As is demonstrated in the following examples, the adhesiveness between a pharmaceutical agent and a bone filling material can be controlled by inactivating a functional group of bone filling material by a blocking agent, and therefore, release of pharmaceutical agent can be controlled.

As later described, the bone filling material, for example, may be tetrapod-shaped, which have pluralities of protruding parts whereon pharmaceutical agent is impregnated or pasted. In this case, pluralities of bone filling materials are to be administered in a defect site. Also similar to an implant, the shape of the bone filling material may be designed considering a patient's bone shape. Generally, the bone filling material is embedded in a patient by a surgical operation, and the material is gradually replaced with bone tissues. The bone filling material may be cement or gelatinous. The bone filling material can be obtained by mixing a calcium-based material, a blocking agent of the calcium-based material, and a pharmaceutical agent (or cement or gelator) as appropriate. The bone filling material can be administered to a defect site as appropriate.

### Calcium-based Material

In general, calcium-based material is the main component of a bone filling material. It is not specifically limited if it is close to bone components. Examples of the calcium-based material include calcium phosphate-based material, calcium carbonate-based material, calcium lactate, and calcium gluconate. Among them, calcium phosphate-based material or calcium carbonate-based material is preferred. Specific examples of calcium phosphate-based material as powdered ingredients include one or more kinds of: hydroxyl apatite, carbonic acid apatite, fluorapatite, chlorapatite, β-TCP, α-TCP, calcium metaphosphate, tetra-calcium phosphate, octa-calcium phosphate, calcium hydrogen phosphate, calcium dihydrogen phosphate, calcium pyrophosphate, the salts thereof, and the solvates thereof. Among them, β-TCP or hydroxyl apatite is preferred. Specific examples of calcium carbonate-based materials include calcium carbonate and calcium hydrogen carbonate. Among them, calcium carbonate is preferred. Chemical compound other than the above may be included in the calcium-based material as needed if the above chemical compound is the main component of the calcium-based material.

Dicarboxylic acid is known to form a compound with an apatite-based compound. (e.g., Hideki Monma "dicarboxylic acid compound apatite system intercalation compound" Gypsum & Lime No .237 (1992) pp .38-44). In the present invention, the calcium-based material may be: a compound of C₃-C₁₀ dicarboxylic acid and one or more kinds of chemical compounds selected from the group consisting of "hydroxyapatite, carbonate apatite, fluorapatite, chlorapatite, β-TCP, α-TCP, calcium metaphosphate, tetra-calcium phosphate, octa-calcium phosphate, calcium hydrogen phosphate, calcium dihydrogen phosphate, calcium pyrophosphate"; salts thereof; or solvates thereof. Among them, a preferred embodiment is a compound of C₃-C₈ dicarboxylic acid and "hydroxyapatite, carbonate apatite, fluorapatite, chlorapatite, β-TCP, α-TCP, calcium metaphosphate, tetra-calcium phosphate, octa-calcium phosphate".

The examples of C₃-C₁₀ dicarboxylic acid include malonic acid, succinic acid, glutaric acid, adipic acid, pimeric acid, suberic acid, azelic acid, sebacic acid, maleic acid, fumaric acid, carbinyl succinic acid, citraconic acid, β-dihydromuconic acid, cis, cis-muconic acid, phthalic acid (benzene -1,2- dicarboxylic acid) or isophthalic acid (benzene -1,3- dicarboxylic acid).

### Blocking Agent

Blocking agent is not specifically limited if it can control the absorption of a pharmaceutical agent to the bone filling material. Preferred blocking agent, however, can bind to a functional group (e.g., hydroxyl group) of the bone filling material by ester binding, amide binding, or the like, and prevent the functional group from binding with a pharmaceutical agent. As described in Patent No. 3758703, Patent No. 3722891, etc., blocking agent suppresses the activity of a functional group temporarily by replacing itself with a predetermined functional group. In order to maintain a certain level of drug release, it is preferred that the binding ability of a blocking agent with a pharmaceutical agent be not too high. In particular, a blocking agent having lower binding ability with a pharmaceutical agent compared to the calcium-based material is preferred (e.g., the binding ability is preferred to be between 1x10⁻² and 0.5 times that of the calcium-based material).

An example of a blocking agent comprises one or pluralities of (e.g., between 2 to 10) groups selected from the group consisting of "carboxyl group, amino group, hydroxyl group, one of the groups represented by the equation (I) below, -NHR¹, -N(R¹)₂, -SH, -R¹, -NHCOR¹, -NHSO₂R¹, -NHCN, -CH (CN), and -SH", wherein the -R¹ represents C₁₋₆ alkyl group, preferably methyl group or ethyl group, and wherein the molecular weight (the number average molecular weight) of the blocking agent is from 75 to 1x10⁵ (preferably between 1x10² and 1x10⁴, more preferably between 1x10² and 1x10³). As is demonstrated in the example below, a chemical compound including a carboxyl group, an amino group, and a hydroxyl group exhibits a preferred blocking ability. Therefore, it can also be considered that a chemical compound having a functional group which has similar function as that of a carboxyl group, an amino group, and a hydroxyl group exhibit a preferred blocking ability. It is known that dicarboxylic acid forms a complex with an apatite-based compound (e.g., Hideki Monma "dicarboxylic acid compound apatite system intercalation compound" Gypsum & Lime No .237 (1992) pp. 38-44). Therefore, it can also be considered that dicarboxylic acid is effective as a blocking agent. Considering the size of the molecule, a preferred dicarboxylic acid as a blocking agent is C₃-C₁₀ dicarboxylic acid. Specific examples of C₃-C₁₀ dicarboxylic acid include malonic acid, succinic acid, glutaric acid, adipic acid, pimeric acid, suberic acid, azelic acid, sebacic acid, maleic acid, fumaric acid, carbinyl succinic acid, citraconic acid, β-dihydromuconic acid, cis, cis-muconic acid, phthalic acid (benzene -1,2- dicarboxylic acid) or isophthalic acid (benzene -1,3- dicarboxylic acid).

Basic structure of a blocking agent is straight-chain, branched-chain or ringed C₁₋₁₀ alkylene group, and may have one or pluralities of the above functional groups. Also, well-known engineering plastics can be adopted as a blocking agent as appropriate. Specific examples of the plastic include: one or a mixture of more than one selected from a group consisting of "acrylonitrile butadiene styrene copolymer, acrylic acid ester rubber, acrylonitrile chlorinated polyethylene styrene copolymer, acrylonitrile ethylene propylene rubber styrene copolymer, amorphous polyalphaolefin, atactic polypropylene, acrylonitrile styrene copolymer, acrylonitrile styrene acrylate, polypropylene, cis-1,4 polybutadiene rubber, cellulose acetate butyrate, cellulose acetate propionate, cresol resin, cresol formaldehyde, carboxymethyl cellulose, nitro cellulose, hydrin rubber, chlorinated polyethylene, chlorination chloroethylene, chloroprene rubber, casein, cellulose triacetate, diallyl phthalate, ethylene chlorotrifluoroethylene copolymer, ethylene diamine tetra-acetic acid, ethylene ethylacrylate, ethylene methacrylic acid, epoxy resin, ethylene propylene diene terpolymer, ethylene tetrafluoro ethylene copolymer, ethylene vinyl acetate copolymer, ethyl vinyl ether, ethylene vinyl alcohol copolymer, fluorinated ethylene propylene, tetra fluoroethylene - hexafluor propylene copolymer, furan resin, flexible urethane foam, fiberglass reinforced plastics, fiberglass reinforced thermoplastic resin, fiberglass reinforced plastics, fiberglass reinforced thermoplastic resin, high density polyethylene, butyl rubber, isophorone diisocyanate, isoprene rubber, methyl methacrylate acrylonitrile buta diene styrene, methyl methacrylate styrene-butadiene rubber copolymer, diphenylmethane diisocyanate, melamine-formaldehyde, methyl methacrylate, melamine phenol formaldehyde, nitrile rubber, expanded polypropylene, polyamide (nylon), polyacrylic acid, polyallyl ether ketone, polyamide-imide, polyester alkyd resin, polyacrylonitrile, polyarylate, polyallyl sulfone, polybutene-1, polybutadiene acrylonitrile, polybenzimidazole, poly - n - butyl methacrylate, polyparaphenylene benzobisoxazole, polybutadiene styrene, polybutylene terephthalate, polycarbonate, polytrifluorochloroethylene, polychlorotrifluoroethylene, polyethylene, polyetherimide, polyether nitril, polyethylene naphthalate, polyethylene oxide, polyethersulfone, polyethylene terephthalate, phenolformaldehyde, tetrafluoroethylene perfluoroalkyl, vinyl ether copolymer, polyimide, polyisobutylene, polymethoxy acetal, polymethacrylonitrile, polymethylmethacrylate (acrylic), polymethylpentene, polyoxymethylene (acetal), polypropylene, polyphthalamide, propylene copolymer, polyphenylene ether, polyphenylene oxide, polyphenylene sulfide, polysulfone, polytrifluorochloroethylene, poly-tetra-fluoroethylene, polytrimethylene terephthalate, polyurethane, polyvinyl alcohol, polyvinyl acetate, polyvinyl butyral, polyvinyl chloride, polyvinyl chloride acetate copolymer, polyvinyl dichloride, polyvinylidene chloride, polyvinylidene fluoride, polyvinylidene fluoroethylene, polyvinyl fluoride, polyvinylformal, polyvinyl isobutyl ether, polyvinyl methyl ether, polyvinyl alcohol, acrylonitrile-styrene copolymer, styrene-butadiene, styrene block copolymer, styrene-butadiene rubber, styrene-butadiene block copolymer, styrene ethylene butylene styrene block copolymer, styrene ethylene propylene styrene block copolymer, silicone, styrene isoprene styrene block copolymer, toluene diisocyanate, tris (nonylphenyl) phosphite, thermoplastic elastomer, methyl pentene, urea-formaldehyole resin, chloroethylene, polyvinyl chloride ethylene,"; a chemical compound having the above basic structure with its one or pluralities of functional groups being replaced with groups selected from the group consisting of "one of the groups described in equation (I) below, -NHR¹, -N(R¹)₂, -SH, -R¹, -NHCOR¹, -NHSO₂R¹, -NHCN, -CH (CN), -CH₂ (CN), -CH (CN)₂, and -SH"; or the mixture thereof (hereinafter, R¹ represents C₁₋₆ alkyl group).

The examples of C₁₋₆ alkyl group include: methyl, ethyl, n-propyl, isopropyl, n-butyl, isobutyl, sec-butyl, tert-butyl, n-pentyl, isopentyl, neopentyl, 1-methylpropyl, n-hexyl, isohexyl, 1,1-dimethylbutyl, 2,2-dimethylbutyl, 3,3-dimethylbutyl, or 3,3-dimethylpropyl.

The examples of blocking agent are one or a mixture of more than two selected from the group consisting of amino acid, peptide, protein, saccharide, glycoprotein, and glycolipid. More specific examples thereof are one or a mixture of more than two selected from the group consisting of amino acid, peptide, protein, or saccharide. In particular, when pharmaceutical agent is oil soluble, the blocking material is preferred to be water soluble, because of low connectivity between the pharmaceutical agent and the blocking agent. From this perspective, when pharmaceutical agent is oil soluble, preferred examples of the blocking agent are amino acid, a deliberative of the amino acid, or pharmaceutically acceptable salt thereof. From this perspective, the examples of the blocking agent are one or a mixture of more than two selected from an amino acid, a derivative of amino acid, or a pharmaceutically acceptable salt thereof, which are selected from the group consisting of "alanine, arginine, asparagine, aspartic acid, cysteine, glutamine, glutamic acid, glycine, histidine, isoleucine, leucine, Lysine, methionine, phenylalanine, proline, serine, threonine, tryptophan, tyrosine, and valine", wherein a carboxyl group may be replaced with one of the groups represented by the formula (I) (in the formula (I) and hereinafter, R¹ represents C₁₋₆ alkyl group), an amino group may be replaced with -NHR¹, -N(R¹)₂, -SH or -R¹, a hydroxyl group may be replaced with -NHCOR¹, -NHSO₂R¹, -NHCN, -CH (CN), -CH₂ (CN), -CH (CN)₂, or -SH. A derivative of amino acid is produced by replacing an amino group or a hydroxyl group of amino acid with the above substituent group. In order to reduce effects on patients, essential amino acids are preferred. The examples of essential amino acids include d-glutamine, 1-glutamine, d-serine, or 1-serine. Among them, 1-serine is desirable.

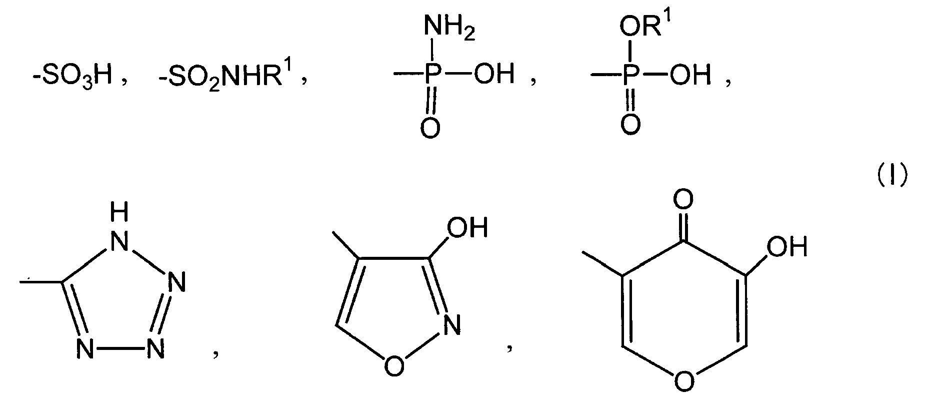

Hereinafter, glycopeptide and glucolipids as blocking agents are explained. The glycopeptide and glucolipids generally comprise one or pluralities of groups selected from the group consisting of "carboxyl group, amino group, hydroxyl group, one of the groups represented by the equation (I) below, -NHR¹, -N(R¹)₂, -SH, -R¹, -NHCOR¹, -NHSO₂R¹, -NHCN, -CH (CN), -CH₂ (CN), -CH (CN)₂, and -SH" and are chemical compounds whose molecular weight (number average molecular weight) is between 75 and 1x10⁵ (preferably between 1x10² and 1x10⁴, more preferably between 1x10² and 1x10³), but one or pluralities of the groups of the molecule may be replaced with "carboxyl group, amino group, hydroxyl group, one of the groups represented by the equation (I) below, -NHR¹, -N(R¹)₂, -SH, -R¹, -NHCOR¹, -NHSO₂R¹, -NHCN, -CH (CN), -CH₂ (CN), -CH (CN)₂, and -SH" as appropriate. Glycoprotein comprises an amino acid with a sugar chain coupled thereto. In the present invention, since a sugar or an amino acid is demonstrated to act as a blocking agent, it can be seen that a glycoprotein can also act as a blocking agent. The represented examples of the blocking agent are: glycoprotein; a asparagines-linked sugar chain (N-linked type), a serine or threonine -linked sugar chain (O-linked type, mucin type), but are not limited to them. The examples of a sugar of glycoprotein include glucose, galactose, mannose, fucose, N-acetylglucosamine, N-acetyl-galactosamine, N-acetylneuramic acid, xylose, and the like. In the present invention, any of the above can be adopted. When the glycoprotein is produced from yeast, however, the preferred sugar of the glycoprotein is mannose. The examples of glycoprotein used for a blocking agent include luteinizing hormone, follicle stimulating hormone, thyrotrophic hormone, human chorionic gonadotropin, avidin, cadherin, proteoglycan, mucin, and lectin. A preferred blocking agent is absorbed by a calcium-based material adequately, and is excellent in biocompatibility. From this perspective, among them, proteoglycan is preferred to be used as a blocking agent. A typical proteoglycan has a core protein coupled with one or pluralities of glycosaminoglycan chains. The examples of proteoglycan are chondroitin sulfate, heparan sulfate, keratan sulfate, and the like. Among them, chondroitin sulfate is preferred as a capping agent. A well-known lectin can be used for a blocking agent. But C-type lectin is preferred for a blocking agent due to the sugar-binding specificities. The glycolipid is a generic designation of materials including both a water-soluble sugar chain and a lipophilicity group in a molecule. The glycolipid is divided into two groups: the sphingoglycolipid whose lipophilicity group is ceramide (V-acylsphingosine); and the glycoglycerolipid whose lipophilicity group is acyl or alkylglycerol. In addition, glycoside having lipophilicity groups such as a steroid (sterylglycoside, steroid glycoside), a hydroxy fatty acid (rhamnolipid) is also included in the glycolipid. A well-known glycolipid can be used as the blocking agent of the present invention, as appropriate.

On the other hand, as is demonstrated in the example below, saccharide such as mucopolysaccharide, disaccharide, and the like, also acts as a preferred blocking agent. Therefore, examples of a blocking agent include one or more kinds selected from a saccharide, a saccharide derivative, or a pharmaceutically acceptable salt thereof, the saccharide being selected from the group consisting of "pentose, hexose, heptose, octose, nonose, decose, maltose, cellobiose, gentiobiose, melibiose, lactose, turanose, sophorose, trehalose, isotrehalose, sucrose, isosaccharose, maltotriose, cellotriose, hyaluronic acid, teichoic acid, colominic acid, chondroitin sulfate, dermatan sulfate, heparin, heparan sulfate, keratan sulfate, chitin saccharide, pullulan, guar gum, lambda carrageenan, traganth gum, pectin, mannan, dextran, and maltodextran", and wherein a hydroxyl group may be replaced with -NHCOR¹, -NHSO₂R¹, -NHCN, -CH₂ (CN), -CH (CN)₂, or -SH. Saccharide deliberative is saccharide with its hydroxyl group substituted with the above substituent. Specific examples of a blocking agent are one or both of serin and dextran.

Pentose, hexose, heptose, octose, nonose, and decose are monosaccharide, and they may be the substitution products such as deoxy sugar, methyl sugar, thio sugar, and amino sugar. Maltose, cellobiose, gentiobiose, melibiose, lactose, turanose, sophorose, trehalose, isotrehalose, saccharose, isosaccharose are disaccharide. Maltotriose, cellotriose are trisaccharide. Hyaluronic acid, teichoic acid, colominic acid, chondroitin sulfate, dermatan sulfate, heparin, heparan sulfate, keratan sulfate are acid mucopolysaccharide, and chitin sugar is neutrality mucopolysaccharide.

Specific examples of polysaccharide thickener used as a blocking agent is one or more kinds selected from the group consisting of pullulan, guar gum, lambda carrageenan, tragacanth gum, pectin, mannan, dextran, malto dextran or acid salts thereof (e.g. sulfate). In order to effectively control the release of a pharmaceutical agent, an example of the average molecular weight (measured by the GPC method) is from 3x10⁵ to 5x10⁷, preferably from 6x10⁵ to 2x10⁷.

A preferred blocking agent is the one that when the pharmaceutical agent is administered in a sufficient amount of water at normal temperature and under normal pressure, the release amount of the pharmaceutical agent within one hour from the administration becomes from 1.5 to 1x10² times (preferably from 2 times to 5 x10 times , more preferably from 3 times to 2 x10 times) in comparison with a case in which a bone filling material without containing the blocking agent is used. In this way, a bone filling material having a preferred drug release can be provided by controlling the release amount as appropriate. The amount of a blocking agent is preferred to be adjusted as appropriate, for example, according to the kinds of a calcium-based material and a pharmaceutical agent. In general, the amount of a blocking agent is from 1x10⁻³ to 1x10¹ times of the total weight of the blocking agent and calcium-based material, preferably from 1x10⁻² to 1x10° times thereof, and more preferably from 5x10⁻² to 5x10⁻¹ times thereof.

The blocking agent is preferably water soluble. The amount of dissolution (solubility) to water 100 g in 20 degrees Celsius is from 1to 2x10² g, preferably from 5 to 1x10² g, and more preferably from 1x10¹ to 7x10¹ g.

The blocking agent may be kneaded in the calcium-based material (this is also included in impregnation), or it may also be impregnated into or pasted to a base material containing the calcium-based material. In particular, in this case, well-known methods such as impregnation application or spray application can be adopted. In the impregnation application, a base material is impregnated in water solution in which the blocking agent was dissolved. The concentration of the blocking agent may be adjusted based on the base material and the blocking agent as appropriate. But, since a functional group of a base material is desired to be blocked appropriately, the concentration is, for example, from 1 to 50 weight percent, and preferably from 2 to 10 weight percent. Since it is enough for the blocking agent to cap all or a part of the functional groups contained in the bone filling material, the molecular weight of the blocking agent may be smaller than that of the calcium-based material. The blocking performance depends on the amount of the blocking agent, so an appropriate amount of the blocking agent is desired to be impregnated into or pasted to the calcium-based material. From this perspective, the specific weight of the blocking agent is, for example, from 1x10⁻³ to 1x10² of that of the calcium-based material, preferably from 1x10⁻² to 1x10¹ thereof, more preferably from 5x10⁻² to 1 thereof, and further preferably from 5x10⁻² to 5x10⁻¹.

When a bone morphogenetic factor (BMP) and myoblast cells such as C2C12 cells are used as a pharmaceutical agent, polysaccharide thickener is preferred to be used as a blocking agent, as demonstrated in the following examples. Noted that the release amount of BMP can be controlled as appropriate by controlling the ratio of the capping by using a blocking agent other than polysaccharide thickener together with polysaccharide thickener as appropriate. Namely, a preferred embodiment of the present invention is to achieve the optimal release of a pharmaceutical agent by combining the above described blocking agents as appropriate in order to control the drug release by using blocking agents.

The pharmaceutical agent, which is contained in the bone filling material of the present invention, is not specifically limited if it has a certain in vivo efficacy. The specific examples of the pharmaceutical agent, later described in detailed examples, is one or more kinds of osteogenesis/chondrongenesis promoter (including chondrongenesis promoting factor), joint disease therapeutic agent, bone/cartilage disease preventive/therapeutic agent, bone regenerating agent, osteoclastic inhibitor, angiogenetic promoter, antibacterial agent, antibiotics, and anticancer agent. In particular, it is, for example, a fibroblast growth factor (FGF) and a bone morphogenetic factor (BMP).

### The Method for Producing Bone Filling Material

Hereinafter, a method for producing a bone filling material is explained. In this method, a base material is formed in the first place, and then, a capping agent and a pharmaceutical agent are impregnated or pasted thereto. So, a method for producing a base material is explained in the beginning. The method for producing a bone filling material having protruding parts, below explained, basically comprises: a kneading step for kneading ingredient which comprises calcium-based material and material containing binder; a molding step for obtaining a molded body having a predetermined shape from a kneaded material obtained in the kneading step with an injection molding machine having a mold; a binder removal (degreasing) step for removing the binder contained in the molded body to obtain a degreased body, the molded body being obtained in the molding step; and a sintering step for heating and sintering the degreased body to obtain a sintered body, the degreased body being obtained in the binder removal step. The method may include publicly known steps such as an after treatment step for a molded body.

Since each of the bone filling material obtained by the production method of the present invention has uniformity in size, appropriate dosage of pharmaceutical agent can be administered, even when the pharmaceutical agent is incorporated in the bone base filling material. Furthermore, the bone filling base materials, when administered, have appropriate porosity while maintaining the strength of each bone filling base material, because the bone filling base material has uniform density and its size can be controllable. Each steps of the method for producing a bone filling base material is explained in the following.

### Kneading Step

In the kneading step, ingredient comprising calcium-based material and material comprising binder are kneaded. In this step, it is preferred to use powdered ingredient. In this step, powdered ingredient and sub materials such as binders are mixed so that they are made to be suitable for injection molding.

### Kinds of Ingredient Powders

Calcium-based materials, for example, are used as powdered ingredients. Examples of the calcium-based materials include calcium phosphate-based materials, calcium carbonate-based materials, calcium lactate, and calcium gluconate. Among them, calcium phosphate-based material or calcium carbonate-based material is preferred. Specific examples of calcium phosphate-based materials as powdered ingredients include one or more than one kind of hydroxyl apatite, carbonic acid apatite, fluorapatite, chlorapatite, β-TCP, α-TCP, calcium metaphosphate, tetracalcium phosphate, calcium hydrogen phosphate, calcium dihydrogen phosphate, calcium pyrophosphate, the salts thereof, and the solvates thereof. Among them, β-TCP or hydroxyl apatite is preferred. Specific examples of calcium carbonate-based materials include calcium carbonate and calcium hydrogen carbonate. Among them, calcium carbonate is preferred. Note that, the powdered ingredients are not specifically limited to these materials, and well-known materials used as ingredients of the bone filling materials can be used as appropriate.

### Size of the Ingredient Powder

When the size of the ingredient powder is too small, a large amount of binders are required for forming a mold, and the physical properties of the resultant bone filling material is deteriorated. On the other hand, when the size of the ingredient powder is too large, there are risks that the ingredient powders get into gaps between a screw and a cylinder of a molding machine, or sintering of ingredient powder does not proceed. In the present invention, powder injection molding is basically performed, but the ingredient powder used in the powder injection molding is not always metal powder. As a result of an experiment, the grain size of the ingredient powder is, for example, from 0.01 µm to 100 µm (both inclusive, same below), and is preferably from 0.1 µm to 20 µm. In a general powder metallurgy, for example, powders with the size of about 100µm are used. For example, in Japanese Patent Laid-Open No. 2004-97259, hydroxyl apatite powder having the grain size of less than 150 µm is used (paragraph [0025]). But in the present invention, it is preferred that ingredient powder having relatively small grain size be used to improve fluidity of kneaded material (mixture of ingredient powders and binders) and improve the density of a sintered body. On the other hand, although the bone filling base material of the present invention requires certain strength, it is assumed to be eroded by osteoclasts. From this perspective, the grain size may be from 0.1 µm to 50 µm, and may preferably be from 0.5 µm to 10 µm.

### Sub Materials

In the kneading step, materials such as binder other than the ingredient (one or more kinds of chemical compound), are mixed with the ingredient. The examples of the binder include (meta) acrylic-based resin, wax lubricant, (preferably, thermoplastic resin other than (meta) acrylic-based resin) and material having lubricant. The example of methacrylic-based resin or acrylic-based resin is methacrylate resin or acrylate resin, and it specifically includes a polymer of n-butyl methacrylate or methyl methacrylate, or a copolymer of n-butyl methacrylate and methyl methacrylate. The molecular weight of methacrylic-based resin or the acrylic-based resin is not specifically limited, but it is preferred to be adjusted as appropriate so that the physical properties of the resultant bone filling material are not lost, and the weight average molecular weight is, for example, 1x10³ to 1x10⁵. The content of methacrylic-based resin or acrylic-based resin in the binders is not specifically limited, but it is preferred to be adjusted as appropriate so that the physical properties of the resultant bone filling material are not lost, and the content is, for example, 1 weight percent to 50 weigh percent.

The example of the wax lubricant is wax having a melting point of 40 to 100 degrees Celsius, and the melting point is preferably from 40 to 70 degrees Celsius. As the wax having the above melting point, for example, well known paraffin wax can be used as appropriate. A molded body can be easily removed from a mold at the time of injection molding by using wax having the above melting point. It is more preferred to use wax having a melting point of 60 to 65 degrees Celsius, because a molded body can be removed from a mold without cooling the mold too much.

The examples of wax lubricant include one or more kinds of: hydrocarbon oil such as liquid paraffin, squalene, and squalane; higher fatty acid such as oleic acid, tall oil, and isostearic acid; higher alcohol such as lauryl alcohol, oleyl alcohol, isostearyl alcohol and the octyldodecanol; silicone oil such as methyl polysiloxane, methyl phenyl polysiloxane, methyl hydrogen polysiloxane, and decamethyl polysiloxane; ester such as isopropyl myristate, isopropyl palmitate, hexyl laurate, oleyl oleate, decyl oleate, octyl dodecyl myristate, hexyl decyl dimethyl octanoate, diethyl phthalate, and dibutyl phthalate; animal and plant oil such as avocado oil, camellia oil, turtle oil, macademia nut oil, corn oil, sesame oil, persic oil, wheat germ oil, sasanqua oil, castor oil, linseed oil, safflower oil, cotton oil, perilla oil, Chinese bean oil, peanut oil, tea seed oil, kaya oil, rice bran oil, jojoba oil, apricot kernel oil, olive oil, carrot oil, grape seed oil, rape seed oil, egg yolk oil, lanolin oil, and mink oil; and glycerine such as glycerine, diglycerol, triglycerine, glycerine trioctanoate, and glycerine triisopalmitate. The melting point of wax lubricant can be controlled by adjusting molecular weight and composition ratio of these raw materials as appropriate.

The molecular weight of wax lubricant is not specifically limited, but it is preferred to be adjusted as appropriate so that the physical properties of the resultant bone filling base material are not lost, and the weight average molecular weight is, for example, from 1x10² to 1x10⁶. The content of wax lubricant in the binders is not specifically limited, but it is preferred to be adjusted as appropriate so that the physical properties of the resultant bone filling material are not lost, and the content is, for example, from 1 weight percent to 50 weight percent.

The examples of a thermoplastic resin include one or more kinds of: polyacetal resin, (meta) acryl resin, polyolefin resin, ethylene-vinyl acetate copolymer, and polyvinyl butyral. However, in the present invention, resins other than (meta) acrylate resin and (meta) acrylic-based resin are preferred to be used as thermoplastic resins. Among them, ethylene-vinyl acetate copolymer is preferred.

The molecular weight of a thermoplastic resin is not specifically limited, but it is preferred to be adjusted as appropriate so that the physical properties of the resultant bone filling base material are not lost, and the weight average molecular weight is, for example, 1x10³ to 1x10⁵. The content of a thermoplastic resin in the binders is not specifically limited, but it is preferred to be adjusted as appropriate so that the physical properties of the resultant bone filling material are not lost, and the content is, for example, from 1weight percent to 50 weight percent.

The examples of lubricant (other than wax lubricant) are stearic acid, a salt of stearic acid, a hydrate of stearic acid, a hydrate of a salt of stearic acid, and C₁-C₅ alkyl stearic acid (C₁-C₅ alkyl represents an alkyl group having 1 to 5 carbon atoms, same below); or one of these materials and polyethylene glycol, or one of these materials and polyglycerol. The content of lubricant in the binders is not specifically limited, but it is preferred to be adjusted as appropriate so that the physical properties of the resultant bone filling base material are not lost, and the content is, for example, 0.5 weight percent to 15 weight percent. The molded bodies can be removed from a mold easily by using the above lubricant. The lubricant may also act as a dispersing agent.

The phthalic acid ester group is another chemical compound comprising the binders. It is reported that the phthalic acid ester group is harmful to the human body. But in a preferred embodiment of the present invention, the binders are thermally decomposed almost completely. So chemical compounds having poor biocompatibility, such as the phthalic acid ester group, can be contained in binders. The example of the phthalic acid ester group is C₁-C₅ alkyl phthalate such as dibutyl phthalate. As demonstrated in the example described below, a bone filling base material having more preferred physical properties could be obtained by consciously using the phthalic acid ester group.

The molecular weight of the phthalic acid ester group is not specifically limited, but it is preferred to be adjusted as appropriate so that the physical properties of the resultant bone filling base material are not lost, and the weight average molecular weight is, for example, from 1x10⁴ to 1x10⁷. The phthalic acid ester group with poor volatility is also preferred. The content of the phthalic acid ester group in the binders is not specifically limited, but it is preferred to be adjusted as appropriate so that the physical properties of the resultant bone filling base material are not lost, and the content is, for example, from 0 weight percent to 20 weight percent, and is preferably from 0.5 weight percent to 15 weight percent.

### Binder Loadings

Binders are removed by being thermally decomposed in the binder removing step described below. The parts where binders existed basically become voids. So the porosity and the intensity of a resultant bone filling base material can be adjusted by controlling the quantity of binders added to ingredient. But in general, the amount of binders required is the amount that is enough to fill spaces between particles of ingredients. This is because if the amount of binders added is not enough, appropriate fluidity can not be obtained, which in turn causes injection molding defects such as short mold and weld, and also causes variations in the shape or the density of the resultant molded bodies. Binder Loadings are, for example, from 10 weight parts to 100 weight parts, based on 100 weight parts of ingredients, or may be 20 weight parts to 50 weight parts. The blending ratio of binders to ingredients is from 25 to 70 volume percent, preferably from 30 to 55 volume percent, and more preferably from 35 to 45 volume percent.

### Addition of Glass Component

A preferred embodiment of the present invention is a method for producing a bone filling base material comprising "ingredient comprising calcium-based material, and material comprising binder", and glass component. As the glass component, the following materials can be used as appropriate: silica glass which is composed mostly of silicon dioxide; borosilicate glass containing 5 to 20 weight percent of B₂O₃; lead glass containing 5 to 40 weight percent of lead; potassium glass containing 5 to 30 weight percent of potassium; Fluoroaluminosilicate glass including sodium fluoride, aluminum fluoride, and the strontium fluoride; or a mixture of one of these glasses and one kind or a mixture of more than one kind of boric acid, lanthanum oxide, gadolinium oxide, niobium oxide, zirconium oxide, and barium. The sinterability of a sintered body is lowered by consciously adding glass components. As a result, minute cracks and porosities are formed on the surface or inside the sintered body, thereby producing a bon filling base material which is suitable for culturing cells. As glass component, the following materials or an appropriate mixture thereof may be used: titanium, titanium alloy, cobalt-chromium alloy, stainless steel, alumina, zirconia. Calcium phosphate-based crystals such as apatite (Ca₁₀ (PO₄)₆ O), or calcium phosphate-based crystals such as CaO-SiO₂-MgO-P₂O₅ based crystallized glass may also be used.

It is preferred that the glass component loadings are adjusted as appropriate based on the physical properties required for the bone filling base material, but the loadings are, for example, between 1 weight parts to 20 weight parts, based on 100 weight parts of ingredients, or may also be 2 weight parts to 10 weight parts. The blending ratio of glass components to ingredients is 1 to 20 volume percent, preferably 2 to 10 volume percent, more preferably 3 to 10 volume percent.

### Addition of Salt or Sugar

A preferred embodiment of the method for producing a bone filling base material of the present invention is to produce a bone filling base material comprising "ingredient comprising calcium-based material, and material comprising binder", and salt or sugar (preferably salt). The sinterability of a sintered body is lowered by consciously adding salt or sugar. As a result, minute cracks and porosities are formed on the surface or inside the sintered body, thereby producing a bone filling material which is suitable for cell culture. And, by immersing the obtained bone filling base material in water, salt or sugar can be removed, thereby producing a bon filling material which is suitable for cell culture. Well-known salts or sugars can be used as appropriate. Salts which can be dissolved in water but is not thermally decomposed at temperature that thermally decomposes binders, particularly inorganic salt, is preferred. Specific examples of the salts include sodium chloride, potassium chloride, calcium chloride or calcium carbonate. Well-known sugars such as sucrose, glucose, and fructose can be used as appropriate. Meanwhile, a preferred embodiment of the present invention comprises thermally-degradable components with or without sugar or salt. The word thermally-degradable component represents a component which is not thermally-degraded in the kneading step, but is thermally-degraded in the molding step and the sintering step, or is thermally-degraded at a higher temperature than the heating temperature of the molding step or the sintering step. Since the thermally-degradable components are thermally-degraded somewhere in the molding step, the sintering step, or after the sintering step, a bone filling base material having appropriate voids can be obtained.

It is preferred that salt or sugar loadings are adjusted as appropriate based on the physical properties required for the bone filling base material, but the loadings are, for example, between 1 weight parts to 20 weight parts, based on 100 weight parts of ingredients, or may be 2 weight parts to 10 weight parts. The blending ratio of salt or sugar to ingredients is 1 to 30 volume percent, preferably 2 to 20 volume percent, more preferably 3 to 10 volume percent. When thermally-degradable components are added to ingredients, the same amount as salt or sugar is preferred to be added.

### Kneading

In the kneading step, compound which is a material for injection molding is obtained by mixing the above described ingredient powders and binders. If ingredient powders are not uniformly mixed, several problems will be caused. For example, the geometry of a molded body will be deteriorated. In particular, it is preferred that each bone filling base material of the present invention have constant geometry in order to maintain the same dosage of pharmaceutical agents. So it is desirable that the ingredients be made as uniform as possible.

The temperature of the kneading step is preferred to be adjusted as appropriate based on the kind of binders. But if the temperature is low, the ingredient powders and the binders can not be mixed, and if the temperature is high, the binders will be thermally decomposed. So the temperature is set to be, for example, from 110 to 240 degrees Celsius , preferably from 130 to 190 degrees Celsius , and more preferably from 140 to 160 degrees Celsius .

In the kneading step, it takes long time for kneading ingredients uniformly. But if the kneading time is too long, the binders will be thermally decomposed. So the kneading time is preferred to be adjusted as appropriate based on the kinds on binders. The kneading time is, for example, from 30 minutes to 5 hours. It may also be from 45 minutes to 1.5 hours.

As a kneading machine used in the kneading step, for example, a pressure type kneader, or a uniaxial or biaxial extrusion kneader can be used as appropriate. Since the bone filling base material of the present invention is pharmaceutical agent which will be used for transplant, it is preferred that the bone filling base material be free of impurities such as broken pieces of kneading blades of a kneading machine. From this perspective, it is desired that the kneading blades be made of material with high hardness, and kneading blades which are provided by surface protective layers (e.g., deposited by TiN coating) are preferred.

A preferred kneading process is, for example, as follows. Firstly, a kneading machine is heated to a predetermined temperature, and binder having high melting point is cast into the kneading machine. When the binder is dissolved to a certain extent, ingredient powders are cast into the kneading machine. After that, binder having low melting point and ingredient powders are cast into the kneading machine, and 1/2 to 4/5 by volume of ingredient is cast into the kneading machine, followed by casting low-volatile components such as DBP (dibutyl phthalate). And then, the remaining ingredient is cast into the kneading machine. In this way, the aggregation of the ingredient powders could be dispersed by kneading binder having high melting point (high-viscous binder) and ingredient powders in the beginning of the step.

In particular, for example, the kneading step comprises the steps of: putting the (meta) acrylic-based resin and the ethylene-vinyl acetate copolymer in a kneading machine; putting the ingredient, the paraffin wax, and the stearic acid in the kneading machine while kneading the (meta) acrylic-based resin and the ethylene-vinyl acetate copolymer; and putting the dibutyl phthalate in the kneading machine while kneading the (meta) acrylic-based resin, the ethylene-vinyl acetate copolymer, the paraffin wax, and the stearic acid. In this way, the compound which is material for injection molding can be obtained.

However, since the bone filling base material of the present invention is replaced with bones in the future, molded products having consciously made minute cracks may be used to promote the replacement. From this perspective, for example, the kneading time may be from 15 minutes to 30 minutes, and the kneading temperature may be from 80 to 100 degrees Celsius.

### Molding Step

The molding step is a process for producing a molded body with a predetermined shape by injection molding. It is preferred that a bone filling base material have four protruding parts extending from the center of the regular tetrahedron form of the bone filling material toward each vertex thereof. Hereinafter an example of a mold for producing the bone filling material is explained. Fig. 1 is a conceptual diagram showing an example of a mold used in the method for producing a bone filling base material of the present invention. Reference number 1 of Fig. 1 represents the shape of the mold with its grooves get together. Reference number 2 represents a part corresponding to an injection hole. Reference number 3 represents a parting face. And reference number 4 represents a wedge. The above described mold, for example, has: a fixed mold having an inlet (gate) where material is injected; and a movable mold which is contacted with the fixed mold when the material is injected, and is apart from the fixed mold after a molded product is formed. And the inlet of the mold is located at a tip part of one of the protruding parts so that the material for injection molding is injected therefrom, and the parting face of the fixed mold and the movable mold is located at the other three protruding parts, wherein the movable mold has ejector pins located inside the other three protruding parts. The term "inside the other three protruding parts" means the direction from the bottom of the Fig. 1(A) to the reference number 2.

The bone filling base material above described has a plurality of protruding parts (e.g., more than four protruding parts). And the mold comprises: a fixed mold having an inlet where material is injected; a movable mold being contacted with the fixed mold when the material is injected, the movable mold being apart from the fixed mold after a molded body is formed, wherein the inlet of the fixed mold is located at a tip part of one of the protruding parts so that the material for injection molding is injected from the tip part of the protruding part, wherein a parting face of the fixed mold and the movable mold has an inclined surface, the inclined surface inclines toward the inlet of the fixed mold from the edge face to the center of the parting face, and wherein the movable mold has dent parts for providing predetermined wedges to the movable mold. As shown in Fig.1, "the parting faces" provided at the parts corresponding to the protruding parts are not horizontally arranged but are inclined toward the inlet of the mold as it extends to the center of the protruding parts.

Producing a bone filling base material with one molding process accompanies difficulties. Those who skilled in the art think of a method for injection molding in which protruding parts independently produced are combined together. But since the bone filling material of the present invention is minute, it is difficult to produce the protruding parts separately and combine them together. The mold of the present invention can produce an injected body having a plurality of protruding parts in one injection molding operation. The mold of the present invention also made it easy for a molded body to be taken out of the fixed mold, for example, by providing a three-dimensional parting face, wherein the parting face of the fixed mold and the movable mold has an inclined surface toward the inlet from the edge to the center of the parting face. For example, when a split mold having a flat parting face is used to mold a molded body, the molded body is highly likely to be stuck in a fixed mold, and it is difficult to remove the molded body from the fixed mold. But the above described mold having inclined parting face lowered the possibility of a molded body remained in a fixed mold.

A preferred embodiment of a mold of the present invention has wedges (4) with the height of 1 µm to 1x10² µm (preferably 5µm to 2x10 µm, or 5µm to 1x10 µm). The shape of the wedge is not specifically limited, if it served as a wedge, and well-known shape of wedge can be adopted. In Fig. 1, three wedges are provided at the parts corresponding to the tip of each protruding part. But the number or the position of the wedges is not particularly limited, and the wedges may be provided at the parts other than the tips of the protruding parts (e.g., the body part of the protruding part). If a molded body remains in the fixed mold, bone filling materials can not be produced. The wedge part serves as a wedge to the moving part, which prevents a molded body being left in the fixed mold when the mold is opened.

A preferred embodiment of the present invention is one of the above described methods for producing a bone filling base material, wherein the movable mold has one or a plurality of grooves (preferably parallel grooves or spiral-shaped grooves) with the depth of 1 µm to 2 x 10 µm (preferably 5 µm to 1 x 10 µm). If a molded body remains in the fixed mold, bone filling materials can not be produced. The groove parts increase the surface area of the movable mold part, thereby preventing a molded body from being left in the fixed mold when the mold is opened. The groove part is provided, for example, on the body part of the protruding parts.

A preferred embodiment of the present invention is one of the above described methods for producing a bone filling base material, wherein the bone filling material has four protruding parts and the movable mold has ejector pins located inside the protruding parts. It is very important for injection molding to remove a molded body from a mold. In this embodiment, a protruding pin is arranged so that it is located inside a protruding part (so that the pin is directed from the other end of the tip of a protruding part through the center of the mold toward the tip of the protruding part.). So a molded body can be taken out effectively.

A preferred embodiment of the present invention is one of the above described methods for producing a bone filling base material, wherein the bone filling base material has a plurality of protruding parts. And the mold comprises: a fixed mold; a movable mold being contacted with the fixed mold when material is injected, the movable mold being apart from the fixed mold after a molded body is formed, wherein an inlet of the material for injection molding is located at a parting face of the fixed mold and the movable mold, wherein the parting face of the fixed mold and the movable mold has an inclined surface, the inclined surface inclines toward the center of the bone filling base material from the edge face to the center of the parting face, and wherein the movable mold has groove parts for providing predetermined wedges to the movable mold. A molded body having a plurality of protruding parts can be obtained in one injection molding operation by an ingenious mold at the timing of injection molding.

In the molding step, an injection molding is performed preferably by using an injection molding machine. The injection molding machine is not specifically limited, and a well-known injection molding machine can be used. The examples of the injection molding machine include: a vertical injection molding machine or a horizontal injection molding machine; a high pressure injection molding machine, a moderate pressure injection molding machine, or a low pressure injection molding machine; or a plunger injection molding machine or a screw injection molding machine. However, in order to produce a bone filling base material having minute protruding parts from calcium phosphate-based material, an injection molding machine which is a horizontal screw type injection molding machine (which is preferably a high pressure injection molding machine) can preferably be used. However, if impurities such as broken pieces of a screw cylinder are mixed in the bone filling material of the present invention, although it will not be a problem for an ordinary molded body, it will be a problem because the bone filling material is intended to be administered in vivo. So it is preferred that a surface protective layer such as a TiN coating layer is preferred to be formed on the surface of the screw.

### Binder Removal Step

In the binder removal step, binder contained in the molded body obtained in the above described molded step is removed, and thereby degreased body is produced. The binder removal step is also referred to as a degreasing step. If binder is not removed sufficiently in the binder removal step, the molded body may be cracked or bloated in the sintering step below. In the degreasing step, it is expected that the binder removal is completed without causing defects on the molded body such as deformations and cracks. The examples of binder removal method include the sublimation method, the natural drying method, the solvent extraction method, the thermal degreasing method, and the like, and the thermal degreasing method is preferred. The thermal degreasing method is performed in an ambient atmosphere, a reduced pressure atmosphere, a pressurized atmosphere, a gas atmosphere, or the like, and is preferably performed in an ambient atmosphere. A molded body is preferably placed on a ceramics setter (which is porous and dense) when it is cast into a degreasing furnace. If the molded body is large (i.e., molded body is thick), porous setter such as alumina setter is preferred. It is also desirable to watch for impurities of contaminated setter and components of a heated setter.

A binder removal step, for example, has several stages of heating-up period and duration period in accordance with the pyrolysis temperature of resin contained in binder. In particular, effective pyrolysis of resin having low pyrolysis temperature improves sintering performance. In the present invention, since temperature is raised as above described, resin having low pyrolysis temperature can be effectively pyrolyzed. A preferred embodiment of the bone filling base material of the present invention may include compounds having poor biocompatibility in binder, although the bone filling base material is administered in vivo. Such compound tends to be a binder having low melting point. So in the heating-up step, in order to vaporize binder having low melting point completely, it is preferred that the temperature be raised relatively moderately. A specific example of heating up ratio is at 1 degrees Celsius/hour to 3x10² degrees Celsius/hour until the temperature reaches in the range of 110 to 300 degrees Celsius which is the temperature of the first maintaining period (preferably until the temperature reaches in the range of 230 to 250 degrees Celsius), preferably at 1x10 degrees Celsius/hour to 2x10² degrees Celsius/hour, more preferably at 2x10 degrees Celsius/hour to 5x10 degrees Celsius/hour, and the ratio may also be at 3x10 degrees Celsius/hour to 4x10 degrees Celsius/hour. The maintaining step is, for example, from 2x10 minutes to 5 hours, preferably from 3x10 minutes to 2 hours.

### Sintering Step

The sintering step is a step for heating a molded body obtained in the binder removal step. Japanese Patent Laid-Open No. 2004-97259 (paragraph [0025]) shows a sintering at 1,250 degrees Celsius for an hour. But in a preferred embodiment of the present invention, a molded body is heated from ambient atmosphere to the highest temperature 9x10² to 1.1x10³ degrees Celsius. This is, for example, to turn α-TCP, as ingredient, effectively into β-TCP. High temperature maintaining period is, for example, 5x10⁻¹ hours to 3 hours. It is noted that in the sintering step, a heating-up step (and maintaining step) is followed by a cooling step, wherein well-known cooling methods are used as appropriate. The sintering period including the cooling period is, for example, from 6 hours to 5x10 hours, preferably from 1x10 hours to 3x10 hours. The molding temperature is, for example, from 1x10² to 1.5x10² degrees Celsius. And the mold temperature is, for example, from 1x10 to 3x10 degrees Celsius.

### Aftertreatment Step

The aftertreatment step is an optional step for aftertreatment of the molded body obtained in the sintering step. Specific examples of the aftertreatment step include patching holes caused by ejector pins, and cleaning the molded body.

It is another preferred embodiment of the present invention to add well-known pharmaceutical agent in addition to ingredient powders. The bone filling base material containing the pharmaceutical agent serves as a proper carrier thereof because the volume of the bone filling base material produced in the present invention is approximately uniform. It is preferred that the pharmaceutical agent added to the bone filling base material retain activity at high temperatures.

### Impregnation and Administration of Sclerosing Solution

It is a preferred method for producing a bone filling material of the present invention to impregnate or paste sclerosing solution to the bone filling base material of the present invention. However, the impregnation and administration of the sclerosing solution is not always an essential step. It is preferred that the sclerosing solution have a function of pre-treating before capping a functional group of the bone filling material by a capping agent as well as a function of adjusting the hardness of the bone filling base material. Specific examples of the sclerosing solution include materials containing polysaccharide or a salt thereof. A preferred example of polysaccharide or a salt thereof contained in the sclerosing solution is mucopolysaccharide or a salt thereof. A specific example thereof is an alkali salt of chondroitin sulfate, and more specific example is sodium chondroitin sulfate. The sclerosing solution may contain pH adjuster such as disodium as needed in addition to polysaccharide. And as solvent, water, purified water, saline, water for injection may be used as appropriate. A bone filling material having preferred adsorption and hardness is desired to be produced. So as is demonstrated in the example, the weight percent of polysaccharide in relation to the entire sclerosing solution is from 1 to 20, and may be from 2 to 10. In the preferred embodiment, after the sclerosing solution is impregnated into or pasted to the bone filling base material, the material is kept wet and then dried. And then the blocking agent is impregnated into and administered to the material. In the wetting step, the wetting period is from 6 hours to 2 days, and it may also be about 12 hours. At the normal temperature, and under vacuum, the material is dried for 24 hours, and obtained a base material. Noted that the drying process may be performed at the high temperature and in low humidity environment.

### Impregnation and Administration of Blocking Agent

The step for impregnating or pasting the blocking agent to the material is not specifically limited if the blocking agent can be impregnated or pasted to the bone filling base material in the step. In the step for producing bone filling base material, the blocking agent may be added as raw material. In particular, the base material is soaked in a solution of the blocking agent, and it is left at rest for from one to six hours at normal temperature. Having the blocking agent impregnated into the base material, the material may go through a pre-drying or a sterilization processing. And then, the bone filling base material, wherein a blocking agent is impregnated into or pasted to, may be dried.

### Impregnation and Administration of Pharmaceutical Agent

The bone filling material of the present invention can be produced by impregnating or pasting a pharmaceutical agent, as appropriate, to the bone filling base material, wherein a blocking agent is impregnated into or pasted to as above described. The methods for administering pharmaceutical agent includes immersion administration, spray administration, and spin coat administration, wherein pharmaceutical composition which is obtained by dissolving pharmaceutical agent with well-known pharmaceutically acceptable diluent (solvent) is administered. Among them, immersion administration is preferred. Immersion administration of pharmaceutical agent impregnates the surface or inside of the bone filling agent with pharmaceutical agent. Namely, the present invention can provide a bone filling material to which a predetermined pharmaceutical agent is impregnated or pasted. The pharmaceutical agent may be pasted to a predetermined site by the ink jet method. In particular, the pharmaceutical agent is administered to a certain designed site by an ink jet device. When the bone filling material is produced by computer-supported design such as rapid prototype, the pharmaceutical agent is preferred to be pasted to the material according to a certain program or input information in the process of producing the bone filling material or bone filling base material.

A preferred embodiment of the bone filling material of the present invention is the above described bone filling material, wherein the pharmaceutical agent comprises an osteogenesis/chondrogenesis promoter (including chondrogenesis promoting factor), a joint disease therapeutic agent, a preventive and/or therapeutic agent for bone/cartilage disease, a bone-regenerating agent, a bone resorption-suppressing substance, an angiogenesis promoter, an antibacterial agent, an antibiotics, or an anticancer agent. A preferred embodiment of the bone filling material of the present invention is the above described bone filling material, wherein the pharmaceutical agent comprises 4,5-dihydro -1 - methyl - 1H- thieno [3,4-g] indazole derivative. 4,5- dihydro -1 - methyl - 1H- thieno [3,4-g] indazole derivative can be produced in accordance with a method disclosed in the Japanese Patent Laid-Open No. 2002-356419. It is desirable that effective dose of the pharmaceutical agent that provides a predetermined efficacy is contained in the bone filling material of the present invention. Namely, since well-known pharmaceutical agent can be used in the present invention, the amount of pharmaceutical agent contained in the bone filling material is preferred to be adjusted as appropriate so that the effective dose of the pharmaceutical agent that provides a predetermined efficacy can be administered.

### Osteogenesis/Chondrogenesis Promoter

As the osteogenesis/chondrogenesis promoter, a publicly known agent for inducing osteogenesis or chondrogenesis can be used as needed. In particular, a chondrogenesis promoter is, for example, a 2-[1-(2,2- Diethoxy-ethyl) -3-(3-p-tolyl-ureido) -2, 3-dihydro-1H-indol-3-yl] -N-p-tolyl-acetamide (2- [1- (2, 2-Diethoxy-ethyl) -3- (3-p-tolyl-ureido) -2,3- dihydro -1H- indol -3- yl] -N-p-tolyl-acetamide]). As a chondrogenesis promoter, for example, osteogenesis promoting factor can be used. The osteogenesis promoting factor is generally referred to as BMP (bone morphogenetic protein). The BMP is a substance for bone/cartilage induction which acts on undifferentiated mesenchymal cells from outside, differentiating them to chondrocyte or osteoblasts. As the osteogenesis promoting factor, for example, BMP1 to 13, or fibroblast growth factor (FGF) such as basic fibroblast growth factor (bFGF) can be used (see Kawaguchi, H., J Clin Endocrinol Metab 86(2): 875-80 (2001)). The BMP used as a pharmaceutical agent of the present invention may be either one of the BMP obtained by genetic recombination or the purified BMP taken form Dunn osteogenic sarcoma (see Takaoka, K., Biomedical Research, 2 (5) 466-471 (1981)). And it can be obtained by a well-known method for producing thereof.

### Joint Disease Treating Agent

Examples of the joint disease treating agent include: anti-inflammatory steroid agents such as p38MAP kinase inhibitor (thiazole-based compound etc., disclosed in WO00/64894), matrix metalloprotease inhibitor (MMPI), prednisolone, hydrocortisone, methylpredinisolone, dexabethamethasone, and bethamethasone; and non-steroidal anti-inflammatory analgesic agents such as indometacin, diclofenac, loxoprofen, ibuprofen, piroxicam, and sulindac.

### Bone/Cartilage Disease Preventing or Treating Agent

Examples of the bone/cartilage disease preventing or treating agent include one or mixtures of more than one kind of the following substances: non-peptide osteogenesis-promoting substances such as prostaglandin A1 derivative, vitamin D derivative, vitamin K₂ derivative, eicosapentaenoic acid derivative, benzylphosphonic acid, bisphosphonic acid derivative, sex hormone derivative, phenolsulfophthalein derivative, benzothiopyran or benzothiepine derivative, thienoindazole derivative, menatetrenone derivative, helioxanthine derivative; and a hardly soluble peptide osteogenesis-promoting substance. These substances can be obtained by a known method. A bone/cartilage disease preventing agent includes one or both of a cartilage disease preventive agent and an agent preventing a bone/cartilage disease from developing.

### Bone-Regenerating Agent

Examples of the bone-regenerating agent include one kind or a mixture of more than one kind of the following substances: calmodulin; actinomycin D; cyclosporin A; glucosamine sulfate; glucosamine hydrochloride; marrow extract; calcium phosphate; lactic acid/ glycolic acid/ ε-caprolactone copolymer; platelet rich plasma; or human marrow mesenchymal cell. These substances can be obtained by a known method.

### Bone Resorption-Suppressing Substance

Examples of the bone resorption-suppressing substance include one kind or a mixture of more than one kind of estrogenic agent, calcitonin, and bisphosphonate. These substances can be obtained by a known method.

### Angiogenesis Promoter

Examples of the angiogenesis promoter include one kind or a mixture of more than one kind of the following substances: indigocarmine; 4- [N-methyl-N-(2-phenylethyl) amino] -1- (3, 5-dimethyl-4-propionyl aminobenzoyl) piperidine; 4-(5H-7, 8, 9, 10- tetrahydro-5, 7, 7, 10, 10-pentamethyl benzo [e] naphtho [2, 3-b] [1.4] diazepine-13-yl) benzoic acid; activated protein C; urotensin II -like peptide compound; fibroblast growth factor (FGF) (including basic FGF and acidic FGF); vascular endothelial cell growth factors (VEGF) (preferably a platelet-derived factor); hepatocyte growth factor (HGF); angiopoetin (including angiopoetin-1 and angiopoetin-2); platelet-derived growth factor (PDGF), Insulin-like growth factor (IGF) or smooth muscle embryo myosin heavy chain(SMemb). Of these substances, fibroblast growth factor is preferred (see Hockel, M. et al., Arch. Surg., No. 128, p. 423, 1993). A basic fibroblast growth factor (bFGF) is preferred as a fibroblast growth factor, and a specific example includes trafermin (gene recombinant). Namely, a preferred embodiment of the bone filling material of the present invention is the above described bone filling material comprising trafermin, a salt thereof, a solvate thereof, or a prodrug thereof. "A salt thereof" represents a salt of trafermin, and is specifically the same salt as above explained. "A solvate thereof" represents a solvate of trafermin, and is specifically the same solvate as above explained. "A prodrug thereof" represents a prodrug of trafermin, and represents an agent that turns into, for example, trafermin, an ion thereof, or a salt thereof in vivo after administration. In particular, a prodrug contains protecting groups such as an amino group which are taken off in vivo, and acts the same as trafermin.

### Antibacterial Agent or Antibiotics

As an antibacterial agent or an antibiotic, a well-known antibacterial agent or antibiotic can be used as appropriate. Specific examples of antibacterial agents or antibiotics include one kind or a mixture of more than one kind as appropriate of the following substances: sulfonamide such as sulfacetamide, sufamethizol, sulfadimidine, and sulfamerazine; chloramphenicols antibacterial agent such as chloramphenicol (CP), and tiamphenicol; quinolones antibacterial agent such as ofloxacin (OFLX), ciprofloxacin (CPFX), enrofloxacin, lomefloxacin (LFLX), rufloxacin, levofloxacin (LVFX), fleroxacin (FLRX), nadifloxacin (NDFX), norfloxacin (NFLX), and sparfloxacin (SPFX); fusidic acid (FA); fusafungine; fosfomycin (FOM), mupirocin (MUP); brodimoprim; dirithromycin; penicillins antibacterial agent such as benzylpenicillin (PCG); penicillin G procaine; benzathine penicillin, phenoxymethylpenicillin (Penicillin V), methicillin, ampicillin (ABPC), cloxacillin (MCIPC), carbenicillin, pivampicillin (PVPC), amoxicillin (AMPC), talampicillin (TAPC), bacampicillin (BAPC), ticarcillin (TIPC), azlocillin, mezlocillin, pivmecillinam (PMPC), piperacillin (PIPC), amoxicillin (AMPC) - clavulanic-acid (CVA) (co-amoxiclav), apalcillin, temocillin, ticarcillin-clavulanic acid (CVA), ampicillin (ABPC) - sulbactam (SBT), sultamicillin (SBTPC), and piperacillin (PIPC) - tazobactam (TAZ); streptomycins antibiotics such as streptomycin (SM); tetracyclines antibiotics such as chlortetracycline, aureomycin, chloramphenicol (CP), oxytetracycline (OTC), demethylchlortetracycline, demeclocycline, ledermycin®, lymecycline, doxycycline (DOXY), and minocycline (MINO); aminoglycosides antibiotic such as neomycin, spectinomycin (SPCM), gentamycin (GM), tobramycin (TOB), amikacin (AMK), micronomicin (MCR), isepamicin (ISP), and arbekacin (ABK); macrolides antibiotics such as erythromycin (EM), spiramycin (SPM), roxithromycin (RXM), azithromycin (AZM), midecamycin (MDM), and clarithromycin (CAM); glycopeptides antibiotics such as vancomycin (VCM), and teicoplanin (TEIC); polypeptides antibiotics such as colistin (CL); streptogramins antibiotics such as virginiamycin, and pristinamycin; lincomycins antibiotics such as clindamycin (CLDM); cephalosporins antibiotics such as cephalexin (CEX), cefazolin (CEZ), cefradine (CED), cefadroxil (CDX), cefamandole (CMD), cefuroxime (CXM), cefaclor(CCL), cefotaxime (CTX), cefsulodin (CFS), cefperazone, cefotiam (CTM), ceftriaxone (CTRX), cefmenoxime (CMX), ceftazidime (CAZ), ceftiroxime, cefonicid, cefpiramide (CPM), cefoperazone (CPZ) - sulbactam (SBT), cefpodoxime (CPDX), cefozidime, cefdinir (CFDN), cefetamet (CEMT), cefpirome (CPR), cefprozil, ceftibufen, and cefepime (CFPM); cephamycins antibiotics such as cefoxitin (CFX), cefmetazole (CMZ), and cefotetan (CTT); oxacephems antibiotics such as latamoxef (LMOX), and flomoxef (FMOX); carbapenems antibiotics such as imipenem (IPM) - cilastatin (CS) (tienam®); monobactams antibiotics such as aztreonam (AZT); carbacephems antibiotics such as loracarbef (LCBF); carbapenems antibiotics such as panipenem (PAPM) - betamipron (BP); ketolides antibiotic such as telithromycin (TEL).

### Anticancer Agent

Anticancer agent is a pharmaceutical agent for treating or preventing cancer. A publicly known anticancer agent can be used as needed. Specific examples of anticancer agent include the following substances: anticancer hemolytic streptococcus formulation such as OK-432 (commercial name Picibanil); anticancer polysaccharide such as krestin, lentinan, schizophyllan, and sonifilan; anticancer antibiotics such as mitomycin C (commercial name Mitomycin, etc.), actinomycin D (commercial name Cosmegen), bleomycin hydrochloride (commercial name Bleo), bleomycin sulfate (commercial name Bleo S), daunorubicin hydrochloride (commercial name Daunomycin), doxorubicin hydrochloride (commercial name Adriacin), neocarzinostatin (commercial name Neocarzinostatin), aclarubicin hydrochloride (commercial name Aclacinon), or epirubicin hydrochloride (commercial name Farmorubicin); mitotic inhibitor such as Vinblastine; alkylating agent such as cis-platin, carboplatin, and cyclophosphamide; antimetabolite such as 5-fluorouracil, cytosine arabinoside and hydroxyurea, N- {5- [N-(3, 4-dihydro-2-methyl-4-oxoquinazoline-6-ylmethyl) -N-methylamino] -2- thenoyl} -L-glutamic acid; anticancer antibiotics such as adriamycin and bleomycin; enzyme such as asparaginase; topoisomerase inhibitor such as etoposide; biological response modifier such as Interferon; antiestrogen such as "NOLVADEX" (tamoxifen); antiandrogen such as "CASODEX"; antimetabolite such as Fluorouracil, Tegafur, Tegafur-uracil, and Methotrexate; plant alkaloid such as Vncristine; anticancer antibiotic such as mitomycin C, actinomycin D, bleomycin hydrochloride, bleomycin sulfate, daunorubicin hydrochloride, doxorubicin hydrochloride, neocarzinostatin, aclarubicin hydrochloride, Aclacinon, and epirubicin hydrochloride; and platinum complex such as cyclotriphosphazene-platinum complex, and cisplatin-platinum complex.

It is expected that the bone filling material of the present invention promotes its replacement with bone tissues in vivo, so pharmaceutical agents including a specific polypeptide or gene may be impregnated into or pasted to the bone filling material. The examples of the polypeptide or the gene include a basic fibroblast growth factor (bFGF), a platelet derived growth factor (PDGF), insulin, an insulin-like growth factor (IGF), a hepatocyte growth factor (HGF), a glial cell line-derived neurotrophic factor (GDNF), a neurotrophic factor (NF), hormone, cytokine (including interleukin, interferon (α, β, or γ), a colony stimulating factor, a tumor necrosis factor, the transforming growth factor), a granulocyte macrophage colony stimulating factor (GM-CSF), granulocyte colony stimulating factor (G-CSF), macrophage colony stimulating factor, neutrophil activating protein, macrophage chemoattractant and activator MCAF, RANTES, macrophage inflammation peptide MIP-1a and MIP-1b, a bone morphogenetic factor (BMP), a vascular endothelial cell growth factor (VEGF). Among them, a growth factor promoting neoangiogenesis and/or osteogenesis is preferred. The examples of the growth factor include a bone morphogenetic factor (BMP), a bone growth factor (BGF), a vascular endothelial cell growth factor (VEGF) and a transforming growth factor (TGF). The specific example is a calponin gene disclosed in the Japanese Patent No. 3713290. The gene is preferred to be contained in the bone filling material as much as the amount which is effective for the gene therapy. It is also preferred that the gene be contained in the bone filling material as it is (naked), in a micelle state, or in the form of a recombinant vector which is transformed into a known vector such as a virus vector. The pharmaceutical agent may be a known genetic antibody.

The gene can be adjusted based on well-known base sequence in accordance with ordinary methods. For example, cDNA of the targeted gene is adjusted in the following method. RNA is extracted from osteoblasts, and primer is produced based on a well-known base sequence, and then cloning by PCR method. Also commercially available genes may be used.

A preferred embodiment of a bone filling material of the present invention is the above described bone filling material including stabilizer. As the stabilizer, a well-known stabilizer used for polymer compound, in particular, pharmaceutically acceptable stabilizing agents can be used as appropriate. The strength of the bone filling material of the present invention is maintained mainly in vivo for extended period. But it is assumed that the bone filling material is decomposed in the early stages due to the existence of enzymes such as protease. So a preferred embodiment of the present invention includes inhibitors such as protease inhibitor. Well-known enzyme inhibitor can be used for the inhibitor as appropriate. The specific examples include one or more than one kind of the following substances: 4-(2- aminoethyl) benzene sulfonyl fluoride, aprotinin, bestain, calpains inhibitor I, calpains inhibitor II, chymostain, 3,4-Dichloroisocoumain, E-64, EDTA, EGTA, lactacystin, leupeptin, MG-115, MG-132, pepstain A, phenylmethyl sulfonyl fluoride, proteasome inhibitor I, p- toluene sulfonyl - L - lysine chloromethylketone, p- toluene sulfonyl - L - phenylalanine chloromethylketone, or tyrosine inhibitor. These protease inhibitors are commercially available, and the inhibitory concentrations thereof are also commonly known. A preferred embodiment of the compound formed by the bone filling material of the present invention maintains the strength for a prolonged period and has sustained drug release. Therefore, the bone filling material of the present invention preferably contains 2 to 100 times of one dosage of the above protease inhibitor, more preferably contains 2 to 50 times thereof. The specific dose level of the protease inhibitor differs based on the kind of the protease inhibitor to be used. The dose preferably contains the amount of protease inhibitor that makes inhibitor's function effective (i.e., the effective dose). In general, the bone filling material (per 1 g) contains 0.1 µg to 0.5 mg of protease inhibitor. The amount included may be 1 µg to 0.1 mg, or may be 10 µg to 0.1 mg. The specific amount of dosage increases in almost proportion to the volume of the site to which the bone filling material is administered.

### Adhesiveness-imparting Agent

Another preferred embodiment of the present invention is a bone filling material (or, a sintered body obtained in the sintering process) to which adhesiveness-imparting agents are impregnated or pasted as appropriate. When a heat-resistant adhesiveness-imparting agent is used, the adhesiveness-imparting agent may be mixed with ingredient powders so that a bone filling material which is powder blended with the adhesiveness-imparting agent can be obtained (in this case, the adhesiveness-imparting agent exists on the surface of the bone filling material, and when the adhesiveness-imparting agent on the surface is replaced with bone tissues, a new surface having the other adhesiveness-imparting agent appears, thereby maintaining the adhesiveness of the bone filling material). Also, a powdered adhesiveness-imparting agent may be sprayed on the surface of a molded body or a sintered body. Furthermore, adhesiveness-imparting agents may be added to the surface of the bone filling material by powder blending, wherein a plurality of bone filling agents and powdered adhesiveness-imparting agents are mixed together and then agitated as needed. The adhesiveness-imparting agent may be impregnated or pasted with the above mentioned pharmaceutical agent, and the adhesiveness-imparting agent alone may be impregnated or pasted. The adhesiveness-imparting agent is an agent for raising adhesion property between the bone filling materials, and it is preferred that the adhesiveness-imparting agent alone do not have high adhesiveness but increase adhesiveness when it contacts with cells in vivo. A specific example of the adhesiveness-imparting agent is Thrombin. Thrombin is one of enzymes which promotes blood clot. Thrombin produces fibrin which is a blood clotting substance in vivo. Fibrin produced by thrombin promotes blood clotting. So when thrombin is used as an adhesiveness-imparting agent, the adhesiveness of the bone filling material will be improved, which raises the strength of the bone filling material by fixing the bone filling materials each other firmly. Thrombin can be impregnated or pasted with the same amount of the above described pharmaceutical agent and in the same manner thereof.

The term "a salt thereof" represents a salt of a compound, particularly represents pharmaceutically acceptable salts of the above described compounds. The term "pharmaceutically acceptable" in this specification means that something is not deleterious to the recipient thereof. The polyphosphoric acid of the present invention can be made to salt by in an ordinary method. The examples of the salt includes: the alkaline metal salts such as sodium salt, potassium salt, and lithium salt; the alkaline earth metal salts such as calcium salt, and magnesium salt; the metal salts such as aluminum salt, iron salt, zinc salt, copper salt, nickel salt, and cobalt salt; the inorganic salts such as ammonium salt; and the organic amine salts such as t-octyl amine salt, dibenzylamine salt, morpholine salt, glucosamine salt, phenylglycine alkyl ester salt, ethylenediamine salt, N-methylglucamine salt, guanidine salt, diethylamine salt, triethylamine salt, dicyclohexylamine salt, N,N-dibenzylethylenediamine salt, chloroprocaine salt, procaine salt, diethanolamine salt, N-benzyl-N-phenethylamine salt, piperazine salt, tetramethylammonium salt, tris (hydroxymethyl) aminomethane salt. Among these salts, as polyphosphoric acid salt, alkaline metal salt is preferred, and sodium salt is more preferred. In this specification, "a salt thereof" may include not only anhydrous salt but also hydrate salt. These pharmaceutically acceptable salts, for example, are ionized in vivo, and act the same as the above described compounds.

The term "a solvate thereof" represents a solvate of a compound. The solvate herein includes a hydrate. The agent of the present invention may absorb moisture, be attached with absorption water, and be hydrated when it is left in the atmosphere or recrystallized. The solvates thus obtained are also included in "a solvate thereof". These solvates are ionized in vivo, and act the same as the above described compounds.

A preferred embodiment of the adhesiveness-imparting agent of the present invention comprises a well known pharmaceutically acceptable diluent as a diluent. Specific examples of the diluent are solvents including one kind or a mixture of more than one kind of water, phosphate buffer solution, citrate buffer solution, phosphate buffered saline or physiological saline solution.

The acid level of the diluent (or solvent) is not specifically limited, but it includes pH 3 to pH 11. In order to improve the strength of the gel obtained to some extent, the acid level is preferably from pH 5 to pH 10, more preferably form pH 6 to pH 9, and further preferably from pH 7 to pH 8. The molar concentration (M) of the diluent is not specifically limited, but it includes 1 mM to 1 M. In order to improve the strength of the gel obtained to some extent, the molar concentration is preferably from 5 mM to 300 mM, more preferably form 10mM to 200mM, and further preferably from 15 mM to 100 mM.

A preferred embodiment of the adhesiveness-imparting agent of the present invention is the above described adhesiveness-imparting agent wherein one or both of the first composition and the second composition include a stabilizing agent. As the stabilizing agent, a publicly known stabilizing agent used for a polymer etc., in particular, a pharmaceutically acceptable stabilizing agent can be used as needed. The compound obtained from the adhesiveness-imparting agent of the present invention is expected to maintain its strength for a prolonged period mainly in vivo. Since enzymes such as protease exists in vivo, the compound may be dissolved by the enzymes. So a preferred embodiment of the bone filling material of the present invention includes inhibitors such as protease inhibitor. As the inhibitors, publicly known enzyme inhibitors can be used as needed. Specific examples of the protease inhibitor include one or more than one kind of 4-(2-aminoethyl) benzene sulfonyl fluoride, aprotinin, bestain, calpains inhibitor I, calpains inhibitor II, chymostain, 3,4-dichloroisocoumain, E-64, EDTA, EGTA, Lactacystin, Leupeptin, MG-115, MG-132, pepstain A, phenylmethyl sulfonyl fluoride, proteasome inhibitor I, p-toluene sulfonyl-L-lysine chloromethylketone, p-toluene sulfonyl-L-phenylalanine chloromethylketone, or tyrosine inhibitor. These protease inhibitors are commercially available, and the inhibitory concentrations thereof are also publicly known. A preferred embodiment of a compound formed by the adhesiveness-imparting agent of the present invention maintains the strength for a prolonged period and has sustained drug release. So the adhesiveness-imparting agent of the present invention preferably contains 2 to 100 times of one dosage of the above protease inhibitor, more preferably contains 2 to 50 times thereof. The specific dose level of the protease inhibitor differs based on the kind of the protease inhibitor to be used. The dose preferably contains the amount of protease inhibitor that makes inhibitor's function effective (i.e., the effective dose). In general, the adhesiveness-imparting agent (1 mL) contains 0.1 µg to 0.5 mg of protease inhibitor. The amount included may be 1 µg to 0.1 mg, or may be 10 µg to 0.1 mg.

When the above described adhesiveness-imparting agent and a pharmaceutical agent is used to form a compound, the resultant compound comprising the adhesiveness-imparting agent contains the pharmaceutical agent, thereby also acting as the bone filling material having sustained drug release. The degradability of the compound can be controlled by adjusting the structure of the adhesiveness-imparting agent and the amount of stabilizing agent added, which makes it possible to adjust the sustained release of the pharmaceutical agent. Namely, the present invention can also provide a method for controlling release property of a pharmaceutical agent by adjusting the amount of the stabilizing agent added to the adhesiveness-imparting agent.

A preferred embodiment of the present invention is provided with a covering layer on the surface of the bone filling agent. The thickness of the covering layer is preferred to be adjusted as needed, but it is for example 1 µm to 5 x 10 µm. And the covering layer includes biocompatible compounds having hydrophilic groups. Examples of hydrophilic group include an OH group, a COOH group, an NH₃ group, a CO₃ group, and a SO₃H group. It is preferred that the covering layer include compounds having an OH group, which is the same hydrophilic group as that of an apatite (Ca₁₀ (PO₄)₆ (OH) ₂) contained in bones because such a covering layer has affinity with bones, thereby promoting osteogenesis. A silica gel layer can be formed on the surface of the bone filling material in the following way. Firstly, acid aqueous solutions such as hydrochloric acid, nitric acid, and sulfuric acid are mixed in liquid glass (Na₂O SiO₂ nH₂O). And when the mixture showed a proper viscosity, the bone filling material is impregnated with the mixture, and then it is taken out from the mixture and is soaked in water so that Na⁺ ions are dissolved in water. The silica gel layer may also be formed in the following way. Firstly, hydrolysis or polymerization reaction of alkoxide of silicon (or titanium) is promoted by mixing alcohol solution of tetramethoxysilane (titanate), tetraethoxysilane (titanate), tetraproxysilane (titanate), tetraisopropoxysilane (titanate), or tetrabutoxysilane (titanate), which are alkoxide of the silicon (or titanium), with catalysers (acid aqueous solution such as hydrochloric acid, nitric acid, sulfuric acid, and acetic acid, or ammonia water solution) as needed. When the mixture showed a proper viscosity, the bone filling material is impregnated with the mixture, and then it is taken out from the mixture.

### Bone Filling Material

The bone filling material obtained by the above method has high dimensional accuracy and exhibits less unevenness. Also, there are less defective materials produced, and mass production of the bone filling material is made possible by the above method. A preferred embodiment of the bone filling material of the present invention preferably has a shape having a plurality of protruding parts. And it is more preferred that the protruding parts are provided so that they are arranged in linear symmetry, plane symmetry, and spatial symmetry. A specific preferred shape of the bone filling material is a tetrapod-shape (a shape having four protruding parts extending toward each vertex from the center of the regular tetrahedron), or a shape having n (n = 6, 8, 12, etc.) protruding parts extending toward each vertex from the center of the regular body having n faces. The size of the bone filling material (the diameter of a sphere that the bone filling material can be accommodated) is, for example, from 1 x 10⁻² mm to 5 mm, preferably from 5 x 10⁻² mm to 3 mm, further preferably from 1 x 10⁻² mm to 2 mm, more preferably from 2 x 10⁻¹ mm to 1.5 mm. Furthermore, a certain disease can be effectively treated and a proper dosage of pharmaceutical agent can be administered by using bone filling material having predetermined pharmaceutical agent pasted on its surface. Hereinafter, preferred shapes of the bone filling material of the present invention are explained.

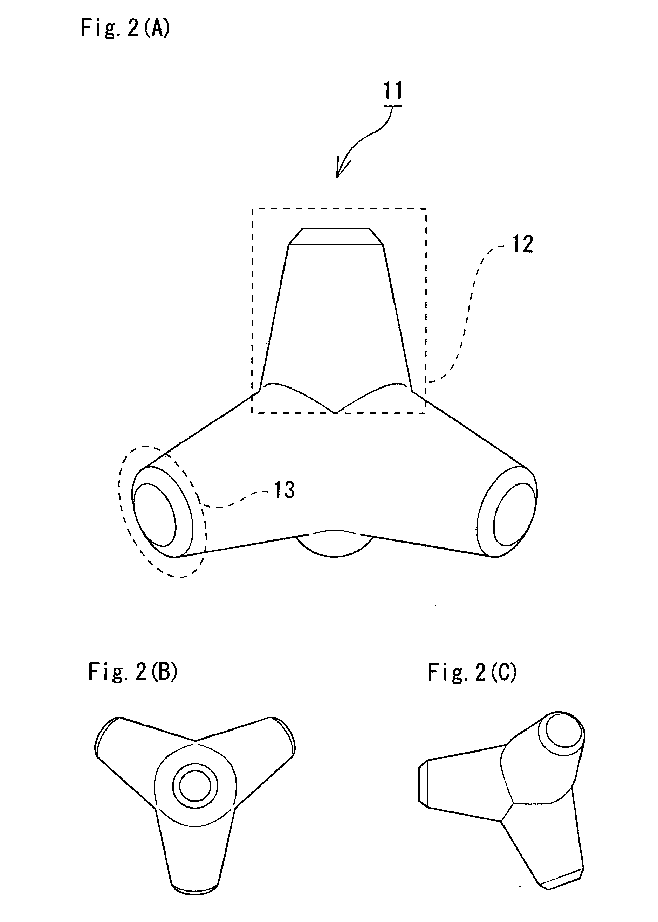

Fig. 2 is a conceptual diagram for explaining an example of a tetrapod-shaped bone filling material. Fig. 2(A) is a side view, Fig. 2(B) is a top view, and Fig. 2(C) is a perspective view. Note that "Fig." means "Figure" (the same below). The bone filling material (11) shown in Fig.2 is a tetrapod-shaped (a shape having four protruding parts (12) extending toward each vertex from the center of the regular tetrahedron) bone filling material. It is preferred that a taper is provided at the tip part (13) of each protruding part (12) and is smoothly shaped (the same below). As shown in Fig.2, each protruding part (12) has substantially the same shape, but one or two of them may be small shaped. Also, each protruding part has, for example, a truncated cone shape which is tapered toward the tip end thereof. The tip portion of each protruding part may be hemispheric (the same below).

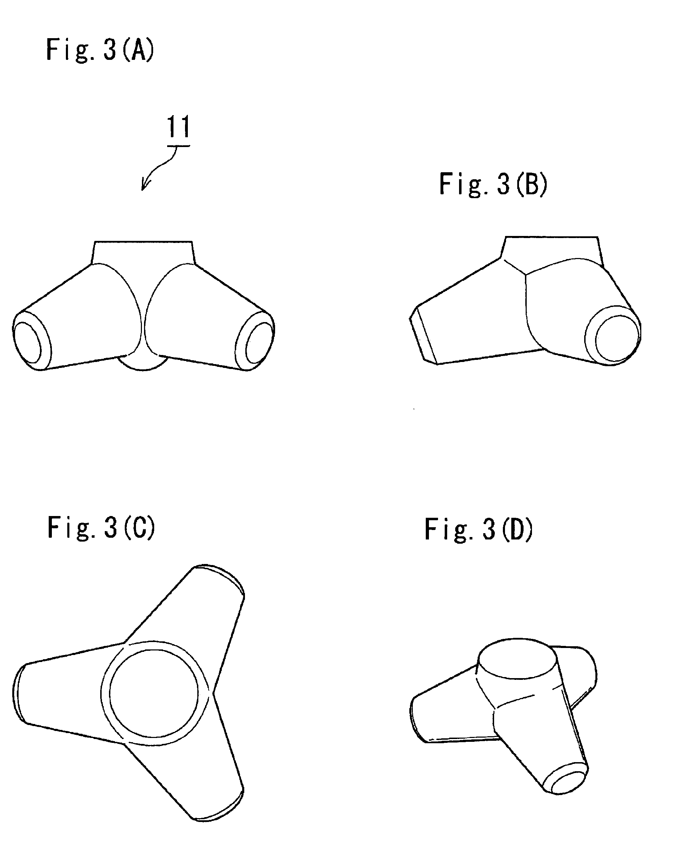

Fig. 3 is a conceptual diagram for explaining an example of a tetrapod-shaped bone filling material with its top portion cut off. Fig. 3(A) and Fig. 3(B) are side views, Fig. 3(C) is a top view, and Fig. 3(D) is a perspective view. The bone filling material according to this embodiment relates to a tetrapod-shaped bone filling material shown in Fig.2 having one protruding part which is cut off in the middle. Specifically, it is a bone filling material having three protruding parts extending toward each vertex from the center of the regular tetrahedron, and a protruding part extending toward the remaining one vertex which is shorter than the other protruding parts. When a plurality of the above shaped bone filling material is used, continuous holes of a cluster of the bone filling material can be made smaller than those of the tetrapod-shaped bone filling material. So the strength of the whole bone filling material can be improved.

The bone filling material produced by the production method of the present invention is injected in bone defect sites, osteoporosis sites, or bone elongation sites. Also, in addition to be filled in the gaps of bones such as bone defect sites, it can also be used as a predetermined carrier of pharmaceutical agents. In this way, the bone filling material can be used for treating or preventing not only bone related diseases but also various other diseases.

### Method for Producing Custom-made Bone Filling Material

Hereinafter, a method for producing a custom-made bone filling material, which is a preferred embodiment of the present invention, is explained. According to the method for producing a bone filling material of this aspect of the present invention, a bone filling base material, which is suitable for each patient, is produced, and then a capping processing is performed on the material, and at the same time, pharmaceutical agent is impregnated into or pasted to the material. In this way, a custom-made bone filling material is produced. In particular, the bone filling agent having a shape which is designed considering a patient's bone shape produced by the rapid prototyping method is preferred because it can be easily produced. A blocking agent or a pharmaceutical agent may be impregnated into or pasted to a bone filling base material which is produced by the rapid prototyping method. Also, a blocking agent or a pharmaceutical agent may be impregnated into or pasted to a bone filling base material in the production process of the rapid prototyping method. However, in the rapid prototyping method, the shape of the bone filling material is designed by a computer and the like, so there is a problem that doctors' viewpoints are not incorporated. Accordingly, it is preferred that a bone filling material be produced from a bone filling base material, which is produced by the method explained below.

### Method for Producing a Bone Filling Base Material

A method for producing a bone filling base material comprises: a digital information of bones obtaining step comprising: a step of photographing a specific part of a patient; and a step of obtaining digital information of bones, the digital information including cross-sectional view of pluralities of the bones, the bones located at the specific part of the patient; a bone model producing step for producing a bone model based on the digital information, the bone model being located at the specific part of the patient, the digital information including cross-sectional view of pluralities of the bones, the digital information being obtained in the digital information of bones obtaining step; a figure-forming material setting step for setting figure-forming material on the bone model produced in the bone model producing step; a figure-forming material digital information obtaining step for obtaining digital information of figure-forming material by photographing the bone model, the bone model on which the figure-forming material being set in the figure-forming material setting step; and a bone filling base material producing step for producing a bone filling base material based on the digital information of figure-forming material, the digital information being obtained in the figure-forming material digital information obtaining step. Namely, steps which are preferred to be performed digitally, such as model forming, bone filling base material producing after figure-forming, and the like are preformed digitally. On the other hand, as described below, an environment where a practitioner's analog skill can be exerted is developed, for example by drawing contour lines on the surface of the bone model, and then the practitioner reflects his analog skill by setting figure-forming material on the bone model based on the contour lines. In this way, highly qualified bone filling base material can be produced.

The step of photographing a specific part of a patient is a step for obtaining digital information of a bone including cross-sectional view of pluralities of the bones by a CT scan or an MRI, the bones being located at the specific part of the patient. And the figure-forming material digital information obtaining step is a step for obtaining the digital information of figure-forming material by a CT scan or an MRI. Namely, pluralities of cross-sectional views of a figure including a bone or figure-forming material can be easily obtained by a CT scan or an MRI. And a three-dimensional digital information of the bone or the figure-forming material can be easily obtained by a computer and the like based on the figures photographed by the CT scan or the MRI. A preferred embodiment relates to one of the above described bone filling base material wherein the specific part of the patient is a site including one of a patient's skull, a lower jaw, an upper jaw, four limbs, or a pelvis. Since these sites include symmetrical parts, deformations can be easily grasped. As a CT imager used for a CT scan and an MRI, a known imager can be used as appropriate. Note that the CT imager is preferred to be connected with a computer. And the computer preferably comprises: an input and output device connected to a CT device and a monitor; a memory for storing image data obtained by a CT or an MRI; a controller (a computing part) for performing arithmetic computation; a main memory storing a program for obtaining three-dimensional digital data of the photographed object part based on pluralities of CT image data or MRI image data; and busses connecting each device together.

A preferred embodiment relates to one of the above described method for producing a bone filling base material wherein the bone model produced in the bone model producing step has contour lines or grid patterns drawn on the surface thereof. If contour lines or grid patterns are drawn on the surface thereof, the practitioner can grasp a bone deformation, a depressed area, and the like quite easily based on the obtained bone model. As a result, quite accurate bone filling base material can be obtained.

A preferred embodiment relates to one of the above described method for producing a bone filling base material wherein the bone model produced in the bone model producing step is a bone model containing gypsum. A preferred embodiment relates to one of the above described method for producing a bone filling base material wherein the bone model producing step is a step for producing a bone model by the rapid prototype method, the injection molding method, the laminate molding method by cutting, or a molding method using processing equipment having a machining center. If a bone model mainly contains gypsum, a bone model can easily be produced by: the rapid prototype method; the injection molding method for producing a bone model using a mold which is designed based on the bone digital information photographed by a CT scan, an MRI, or the like; a molding method using a processing equipment having a machining center based on the obtained bone digital information; a molding method using an NC controllable cutting equipment having a multiple-spindle drilling machine based on the obtained bone digital information. The laminate molding method by cutting is, for example, performed in the way disclosed in JP-A 08-290347 that is "a laminate molding method, wherein a three-dimensional shape is divided into several layers, then each layer is formed and laminated sequentially, comprising the steps of: casting a plate material into three-dimensional curved shape by tools such as a cutting tool or a grinding tool based on the instruction of a three-dimensional processing program which is made for each layer based on three-dimensional numerical data; laminating unprocessed plate material on this processed plate material; and then repeating the step of casting an unprocessed plate material into three-dimensional curved shape by the above tools". Or it may be a laminate molding method for laminating three-dimensional shaped object into two-dimensional half shape by cutting sheet shaped material based on two-dimensional shaped data of each layer of the object, then sequentially laminating the sheet, which is disclosed in JP-A 03-244510 and JP-A 05-313584. Or it may be a laminate molding method for laminating three-dimensional shaped object into two-dimensional half shape by irradiating light beam on the surface of liquid light hardening resin, forming a hardened layer having a predetermined shape, further providing unhardened liquid light hardening resin on this hardened layer and again irradiating light beam, repeating this process and laminating a new layer on the hardened layer. Also, as a machining center, a well known machining center can be used as appropriate. For example, a machining center disclosed in JP-A 2004-074376, JP-A 2003-94264, or JP-A 2001-150262 can be used as appropriate. Also, as a multi spindle drilling machine (e.g., five axis spindle drilling machine) the ones disclosed in JP-A 2006-5257, JP-A 2001-230223, or JP-A 2000-176715 can be used as appropriate.

A preferred embodiment relates to one of the above described method for producing a bone filling base material wherein the bone model produced in the bone model producing step contains calcium-based material and polyvinyl alcohol resin, and wherein the polyvinyl alcohol resin is 2 to 8 weight parts when the total weight of the calcium-based material and the polyvinyl alcohol resin is 100 weight parts. This bone model can be obtained in particular by the injection molding quite quickly and highly accurately. As a result, bone materials can be produced with accurate. A preferred embodiment relates to one of the above described method for producing a bone filling base material wherein the bone model produced in the bone model producing step is made of material containing α-type hemihydrate gypsum and polyvinyl alcohol resin, and wherein the polyvinyl alcohol resin is 2 to 8 weight parts when the total weight of the calcium-based material and the polyvinyl alcohol resin is 100 weight parts.

A preferred embodiment relates to one of the above described method for producing a bone filling base material wherein the bone model contains gypsum as the main ingredient, and wherein the figure-forming material contains wax or plastic more than 90 weight parts per 100 weight parts of the total amount. A preferred embodiment relates to one of the above described method for producing a bone filling base material wherein the bone model contains gypsum as the main ingredient, and wherein the figure-forming material contains wax more than 90 weight parts per 100 weight parts of the total amount. A preferred embodiment relates to one of the above described method for producing a bone filling base material wherein the bone model contains gypsum as the main ingredient, wherein the figure-forming material contains wax more than 90 weight parts per 100 weight parts of the total amount, and wherein the figure-forming material contains titanium oxide of the rutile type of 2 to 5 weight parts per 100 weight parts of the total amount. Namely, when the bone model having this composition and figure-forming material are photographed by a CT scan or an MRI, a bone model portion and a figure-forming material portion can be precisely analyzed, thereby producing a bone filling base material with high accuracy.

A preferred embodiment relates to one of the above described method for producing a bone filling base material wherein the bone filling base material producing step is a step for producing a bone filling base material by the rapid prototype method. A custom-made bone filling base material can be produced quickly and accurately by the Rapid Prototype Method.

A preferred embodiment relates to one of the above described method for producing a bone filling base material, wherein the bone filling base material obtained in the bone filling base material producing step is produced from one or more than one of hydroxyapatite, carbonate apatite, fluorapatite, chlorapatite, β-TCP, α-TCP, calcium metaphosphate, tetra-calcium phosphate, octa-calcium phosphate, calcium hydrogen phosphate, calcium dihydrogen phosphate, calcium pyrophosphate, salts thereof, or solvates thereof. For example, when a bone filling base material is produced from these materials by the injection molding method, phase change occurs at the time of molding, turning it into a bone filling base material having preferred characteristics.

### Method for Producing Bone Filling Base material II

Hereinafter, the other method for producing base material is explained. The method for producing a bone filling base material of the present invention basically comprises: a bone model producing step (step 1) for producing a bone model (1); a figure-forming material filling step (step 2) for filling figure-forming material (3) into a bone defect site (2) of the bone model obtained in the bone model producing step; and a bone filling base material producing step (step 3) for producing a bone filling base material (4) which is filled in the bone deficit cite based on the figure-forming material filled in the bone deficit site of the bone model in the figure forming material filling step. And by a treatment of filling this bone filling base material, the bone deficit site is recovered and cured.

### Bone Model Producing Step (Step 1)

The bone model producing step is a step for producing a bone model. Basically, the bone model is not specifically limited if it is used for producing a patient's bone model, and a well known method for producing a bone model can be used as appropriate. The examples of the method for producing a bone model include: a method for producing a bone model by pouring raw material into a mold which is produced based on, for example, an X-ray image of a bone; and a method for producing a bone model by the rapid prototype method. A method for producing a bone model by the rapid prototype method is preferred, because the bone model is produced based on digital data.

In recent years, rapid prototyping apparatus for easily forming three-dimensional structures (shown in, for example, JP-T 2001-524897, JP-T 2003-531220, JP-T 2004-538191, JP-T 2005-503939, U.S. Pat. No. 5204055 specification, U.S. Pat. No. 5340656 specification, U.S. Pat. No. 5387380 specification, U.S. Pat. No. 6007318 specification, U.S. Pat. No. 6375874 specification, U.S. Pat. No. 5902441 specification and U.S. Pat. No. 6416850 specification. These are incorporated herein for reference purposes) and rapid prototype process are increasingly used. Three dimensional objects, such as prototype parts of apparatus, are used to examine the performance thereof. The examples of rapid prototype method include the stereolithgraphy method, the powder sintering method, the powder binding method, and the solid ground curing technology (SGC), which forms thin layers based on a cross sectional shape data to form three-dimension shape by laminating the thin layers.

In the present invention, a method which is based on the RP method modified as needed for producing a bone model is preferred to be adopted. In particular, a bone filling base material producing method comprising: a cross-sectional shape obtaining step for obtaining information on the cross-sectional shape of each layer by dividing three-dimensional shape of a patient's bone into multiple layers; a first cross-sectional figure forming step comprising the steps of: reading out information on the cross-sectional shape of a first layer from the information on the cross-sectional shape obtained in the cross-sectional shape obtaining step; and forming the first cross-sectional figure duplicating the cross-sectional shape by using a figure-forming composition based on the read out information; a second cross-sectional figure forming step comprising the steps of: reading out information on the cross-sectional shape of a second layer from the information on the cross-sectional shape obtained in the cross-sectional shape obtaining step, the second layer being located on the upper layer of the first cross-sectional figure; and forming the second cross-sectional figure so as to be overlapped with the first cross-sectional figure, the second cross-sectional figure duplicating the cross-sectional shape from a figure-forming composition based on the read out information; and a three-dimensional figure obtaining step for obtaining the three-dimensional figure duplicating the shape of the object, wherein the three-dimensional figure obtaining step repeats, an upper layer cross-sectional figure forming step for forming an upper layer cross-sectional figure, in the same way as the second cross-sectional figure forming step, of: reading out information on the cross-sectional shape of the layer to be formed from the information on the cross-sectional shape obtained in the cross-sectional shape obtaining step; and forming the cross-sectional figure of the layer so as to be overlapped with the cross-sectional figure obtained in the former cross-sectional figure forming step, the cross-sectional figure of the layer duplicating the cross-sectional shape from a figure-forming composition based on the read out information, wherein at least one or more of the cross-sectional figure forming steps comprise: a figure-forming composition layer obtaining step for forming figure-forming composition layers by stratifying powders of the figure-forming composition; and a water adding step for moistening a predetermined part of the figure-forming composition layer by adding water to the figure-forming composition layer based on information on the cross-sectional shape of the layer, the layer formed in the figure-forming composition layer obtaining step.

A well known figure-forming composition can be used as appropriate. But a preferred example of the figure-forming composition is a figure-forming composition which is a mixture of calcium-based material and polyvinyl alcohol resin, wherein the polyvinyl alcohol resin is 2 to 8 weight parts when the total weight of the calcium-based material and the polyvinyl alcohol resin is 100 weight parts.

A preferred embodiment of the figure-forming composition is a figure-forming composition comprising a calcium-based material, a polyvinyl alcohol resin, and a hardening accelerator, wherein the hardening accelerator is one or more kinds of hardening accelerators selected from the group consisting of "dihydrate gypsum, alkali metal sulfate, alkaline earth metal sulfate, alkali metal chloride salt, alkaline earth metal chloride salt, inorganic acid ammonium salt, and alums", wherein the polyvinyl alcohol resin is 2 to 8 weight parts and the hardening accelerator is 0.1 to5 weight parts when the total weight of the calcium-based material and the polyvinyl alcohol resin is 100 weight parts. It is preferred that the figure-forming composition according to this embodiment exclusively comprise calcium-based material, polyvinyl alcohol resin, and hardening accelerator.

It is preferred that these figure-forming compositions do not practically contain water except crystal water, and the one in powdered shaped is preferred. As far as figure-forming composition for building material is concerned, particle size of gypsum powder, which is raw material of the figure-forming composition, will not be a problem because gypsum powder is dissolved sufficiently by being mixed with water and the like. But, since the figure-forming composition of the present invention is not always intended to be in the form of slurry, the particle size of calcium-based material powder is preferred to be almost equalized. From this perspective, equal to or more than 50 weight percent of the molecule of the calcium-based material of the present invention should be located in the range of plus/minus 10 percent of the maximum distribution, according to a measurement of particle distribution based on JISR1619 (Testing method for size distribution of fine ceramic particles by liquid photosedimentation method). It is preferred that equal to more than 70 weight percent, more preferably equal to more than 85 weight percent, and further preferably equal to more than 95 weight percent thereof be located in the range of plus/minus 10 percent of the maximum distribution. This distribution can be achieved by repeatedly sorting out the ingredient powders.

A preferred example of calcium-based material of the figure-forming composition is gypsum. And examples of gypsum include α-type hemihydrate gypsum, β-type hemihydrate gypsum, or mixture of both. Among them, α-type hemihydrate gypsum is preferred. This is because, compared with β-type hemihydrate gypsum, α-type hemihydrate gypsum can achieve a kneaded state with little water, and hardening can be promoted. Hemihydrate gypsum having a small repose angle (repose angle is the maximum angle of inclination at which powders can form a stable slope) is preferred to be used, because the powders can be spread uniformly at the time of molding. From this perspective, the repose angle of hemihydrate gypsum (or figure-forming composition) is in the range of 30 to 45 degree, preferably 35 to 40 degree.

The polyvinyl alcohol resin of the present invention is not specifically restricted, and publicly known polyvinyl alcohol resin (polyvinyl alcohol (-[C(OH)HCH₂]ₙ-) or polyvinyl alcohol resin having a functional group as appropriate) can be used as needed. As the polyvinyl alcohol resin, saponified material (which is produced by saponifying lower alcohol solution of polyvinyl acetate with saponifying catalyst such as alkali or acid, in general) or derivative thererof can be used. Also, as polyvinyl alcohol resin, monomer which copolymerized with vinyl acetate, and saponified material which is a copolymer with vinyl acetate can be used. The examples of monomer which copolymerized with vinyl acetate include: olefine such as ethylene, propylene, isobutylene, α-octene, α-dodecen, and α-octadecene, unsaturated acids such as acrylic acid, methacrylic acid, crotonic acid, maleic acid, maleic anhydride, and itaconic acid or the salt thereof, or monoalkyl or dialkyl ester, nitriles such as acrylonitrile or meta acrylonitrile, amides such as acrylic amide, and methacrylamide, olefine sulfonic acid such as ethylene sulfonic acid, allyl sulfonic acid, and meta allyl sulfonic acid or the salt thereof , alkyl vinyl ethers, N-acrylic amide methyl trimethyl ammonium chloride, allyl trimethyl ammonium chloride, dimethyl diallyl ammonium chloride, dimethyl allyl vinyl ketone, N-vinyl pyrrolidone, vinyl chloride, vinylidene chloride, polyoxyalkylene (meta) allyl ether such as polyoxyethylene (meta) allyl ether and polyoxypropylene (meta) allyl ether, polyoxyalkylene (meta) acrylate such as polyoxyethylene (meta) acrylate and polyoxypropylene (meta) acrylate, polyoxyalkylene (meta) acrylic amide such as polyoxyethylene (meta) acrylic amide, and polyoxypropylene (meta) acrylic amide, polyoxyethylene (1- (meta) acrylic amide -1, 1- dimethylpropyl) ester, polyoxyethylene vinyl ether, polyoxypropylene vinyl ether, polyoxyethylene allylamine, polyoxypropylene arylamine, polyoxyethylene vinyl amine, polyoxypropylene vinyl amine. Preferably, saponified material of homopolymer of the vinyl acetate, saponified substance of copolymer of vinyl acetate and ethylene, unsaturated acid or the salt thereof, alkyl ester and olefine sulfonic acid or the salt thereof are used.

The figure-forming composition of the present invention, different from building materials and the like, is not needed to be placed in a mold nor kneaded. So, saponification degree and average degree of polymerization of polyvinyl alcohol resin is not specifically restricted. On the other hand, since mechanical strength of a three-dimensional figure is not improved with less than 70 mol percent of saponification degree, the saponification degree is preferred to be equal to or more than 70 mol percent, and more preferably it is in the range of 80 to 99.5 mol percent. If the average polymerization degree of polyvinyl alcohol resin is below 2x10², the viscosity of slurry becomes too low. In contrast, if the average polymerization degree of polyvinyl alcohol resin is over 3x10³, the viscosity of slurry becomes too high, which makes it difficult to be dissolved in water. So, the range of polymerization degree is for example 2x10² to 3x10³, and it may also be 5x10² to 2.5x10³. It may also be, for example, 3x10³ to 1x10⁴, because the figure-forming composition of the present invention is not needed to be placed in a mold nor kneaded. Also, if the polymerization degree is low, when water is added to the figure-forming composition and make it in the form of slurry, gypsum particles are settled out therein. But the figure-forming composition of the present invention is not need to be in the form of slurry. Since it is preferred that the polymerization degree be low and be easily dissolved in a little amount of water, the polymerization degree is, for example, 5x10 to 1.9x10², and it may also be 1x10² to 1.5x10². The polymerization degree or molecular weight can be controlled by adjusting reaction time or conditions as appropriate based on publickly known method.

Concerning polyvinyl alcohol resin, saponification degree of complete saponification type is, for example, in the range of 90 to 99.5 mol percent both inclusive. And more preferably the range is 98.5 to 99.5 mol percent both inclusive. As for viscosity thereof, 1x10 to 2x10 mPa·s is preferred. The viscosity is preferred to be measured based on JIS standard (e.g. JIS K 7367).

Note that polyvinyl alcohol resin may be polyvinyl alcohol resin itself, and it may be the resin of the polyvinyl alcohol derivative introducing a functional group as appropriate. Also, the functional group may be partially introduced thereto. And the polyvinyl alcohol resin may be a mixture of several kinds of polyvinyl alcohol resin. The examples of the functional group include an acetoacetyl group, a silyl group, a quaternary ammonium base, a carboxylic acid group, an inorganic base of carboxylic acid, a sulfonic group, an inorganic base of the sulfonic acid, a ketone group, a mercapto group, and an amino group. One or more than one kind of the above functional groups may be included. Among them, an acetoacetyl group or a silyl group is preferred, and the most preferred one include an acetoacetyl group as a functional group. Note that all the hydroxyl groups (-OH) may be substituted with functional groups, 5 to 95% of the hydroxyl groups may be substituted with functional groups, and 10 to 20%, 70 to 90%, or 30 to 70% of the hydroxyl groups may be substituted with functional groups. In particular, polyvinyl alcohol resin having an acetoacetyl group forms chelate with a metal ion which is contained in hardening accelerator, thereby achieving a prescribed hardness with little amount of water in a short period. These functional groups can be introduced to polyvinyl alcohol resin obtained as appropriate, based on a general method of organic synthesis. The kind or ratio of functional groups introduced can also be controlled based on a general method of organic synthesis.

The polyvinyl alcohol resin is mixed with the calcium-based material so that the polyvinyl alcohol resin is 2 to 8 weight parts per 100 weight parts of the total of the calcium-based material and the polyvinyl alcohol resin. As demonstrated in the example below, polyvinyl alcohol resin is preferred to be in the range of 3 to 7 weight parts. It may also be in the range of 3 to 6 weight parts, or 4 to 7 weight parts. It may further be in the range of 4 to 6 weight parts, or 4.5 to 5.5 weight parts. A suitable level of hardness can not be achieved with little amount of polyvinyl alcohol resin.

The figure-forming composition of the present invention may include the polyvinyl alcohol resin which is separate from calcium-based material, or it may be a mixture of calcium-based material and polyvinyl alcohol resin. In both cases, the figure-forming composition is preferred to be in a powdered state, and the size of the powders are preferred to be in the range as above described.

The hardening accelerator of the present invention is one or more than one kind of hardening accelerator selected from the group consisting of: dihydrate gypsum, alkali metal sulfate, alkaline earth metal sulfate, alkaline metal chloride salt, alkaline earth metal chloride salt, inorganic acid ammonium salt, and alums. The examples of alkali metal sulfate include sodium sulfate and potassium sulfate. The examples of alkali earth metal sulfate include magnesium sulfate, calcium sulfate and barium sulfate. The examples of alkali metal chloride salt include lithium chloride, sodium chloride and potassium chloride. The examples of alkaline earth metal chloride salt include magnesium chloride and calcium chloride. The example of inorganic acid ammonium salt includes ammonium hydrochloride. The examples of alums include potassium alum such as aluminum potassium sulfate 12 water: AlK (SO₄)₂·12H₂O_{,} sodium alum such as AlNa (SO₄)₂·12H₂O, ammonium alum such as NH₄Al (SO₄)₂·12H₂O. Among them, one or more than one kind selected from the group consisting of magnesium sulfate, sodium chloride, sodium sulfate, and calcium sulfate can preferably be used. And, a mixture of dihydrate gypsum; and one kind or more than on kind selected from the group consisting of magnesium sulfate, sodium chloride, sodium sulfate, and calcium sulfate can preferably be used. Also, a hardening accelerator having metal salt is preferred, because it forms a chelate structure with polyvinyl alcohol having a predetermined functional group, and improves the hardness of three-dimensional figures or three-dimensional structures.

When the hardening accelerator is added to the mixture of calcium-based material and polyvinyl alcohol resin, 0.1 to 5 weight parts of the hardening accelerator is preferred to be added to 100 weight parts of the total of calcium-based material and polyvinyl alcohol resin. The amount of dihydrate gypsum as hardening accelerator is, for example, 0.5 to 5 weight parts. On the other hand, the amount of hardening accelerator contained not having dihydrate gypsum is, for example, 0.1 to 5 weight parts, preferably 0.1 to 3 weight parts, further preferably 0.3 to 2 weight parts, and more preferably 0.4 to 1.5 weight parts, per 100 weight parts of the total amount of hemihydrate gypsum and polyvinyl alcohol resin.

The hardening accelerator is preferred to be mixed with figure-forming composition according to a publicly-known method in the field of figure-forming composition. The figure-forming composition of the present invention may include known compositions other than the ones above described as far as it retains the function of the figure-forming composition of the present invention.

In the method for forming a three-dimensional figure used for forming a bone model, basically, one of the above described figure-forming compositions which is in powdered state is used, when a three-dimensional figure is produced according to the rapid prototype process (the RP process). By using the above described figure-forming composition, even if a three-dimensional figure is formed by accumulating multiple layers to which a little amount of water (water, cross-linker solution, publicly known binder aqueous solution used for RP apparatus) was added, a three-dimensional figure having enough hardness to maintain a tentative form can be formed in a short period. It is also preferred that a unified three-dimensional figure be formed from adhesively joined layers each of which have certain level of hardness by being added with a small amount of water. The three-dimensional figure having the above characteristics can not be obtained by using the existing figure-forming composition as it is. But, by using the figure-forming composition of the present invention, a method for forming a three-dimensional figure according to this aspect can be obtained.

In particular, the method for forming a three-dimensional figure used for forming a bone model is a method for forming a three-dimensional figure duplicating the shape of an object, the method comprising: a cross-sectional shape obtaining step (step A1) for obtaining information on the cross-sectional shape of each layer by dividing three-dimensional shape of the object into multiple layers; a first cross-sectional figure forming step (step A2-1) comprising the steps of: reading out information on the cross-sectional shape of a first layer from the information on the cross-sectional shape obtained in the cross-sectional shape obtaining step; and forming the first cross-sectional figure duplicating the cross-sectional shape by using a figure-forming composition based on the read out information; a second cross-sectional figure forming step (step A2-2) comprising the steps of: reading out information on the cross-sectional shape of a second layer from the information on the cross-sectional shape obtained in the cross-sectional shape obtaining step, the second layer being located on the upper layer of the first cross-sectional figure; and forming the second cross-sectional figure so as to be overlapped with the first cross-sectional figure, the second cross-sectional figure duplicating the cross-sectional shape from a figure-forming composition based on the read out information; a three-dimensional figure obtaining step (step A2-n) for obtaining the three-dimensional figure duplicating the shape of the object, wherein the three-dimensional figure obtaining step repeats, an upper layer cross-sectional figure forming step for forming an upper layer cross-sectional figure, in the same way as the second cross-sectional figure forming step of: reading out information on the cross-sectional shape of the layer to be formed from the information on the cross-sectional shape obtained in the cross-sectional shape obtaining step; and forming the cross-sectional figure of the layer so as to be overlapped with the cross-sectional figure obtained in the former cross-sectional figure forming step, the cross-sectional figure of the layer duplicating the cross-sectional shape from a figure-forming composition based on the read out information, wherein at least one or more of the cross-sectional figure forming steps comprise: a figure-forming composition layer obtaining step for forming figure-forming composition layers by stratifying one of the powders of the figure-forming composition above described; and a water adding step for moistening a predetermined part of the figure-forming composition layer by adding water to the figure-forming composition layer based on information on the cross-sectional shape of the layer, the layer formed in the figure-forming composition layer obtaining step. Hereinafter, each step is explained.

A cross-sectional shape obtaining step (step A1) is a step for obtaining information on the cross-sectional shape of each layer by dividing three-dimensional shape of the object into multiple layers based on information on the three-dimensional shape of the object. A preferred embodiment of the method for forming a three-dimensional figure used for forming a bone model is the above described method for forming three-dimensional figures, wherein the information on the cross-sectional shape of each layer comprises color identification information of each layer, and wherein water including coloring component is added based on the color identification information in the water adding step.

The method for forming three-dimensional figures used for forming a bone model can be easily performed by a publicly known apparatus used in, what is called, the rapid prototype process, which is programmed to perform the steps of the method. In particular, the method is performed easily by using a computer which is programmed for the rapid prototype process. This computer comprises an input/output part, a control part such as a CPU, a computing part, and a memory part. And it is connected with three-dimensional figure forming part for forming three-dimensional figures via an input/output part such as an interface. And the three-dimensional figure forming part comprises: a movable table for moving upward and downward to form a three-dimensional figure based on directions from the computer; a figure forming composition layer forming part for forming figure forming composition layer, by taking out figure forming composition powers from figure forming composition powder storing part in order to form figure forming composition layers on the movable table based on the orders from the computer; and a printing part for adding water or prescribed aqueous solution to the figure forming composition layer based on the orders from the computer.

In the cross-sectional shape obtaining step, it is preferred to obtain information concerning three-dimensional shape of an object, and then obtain images divided in cross-sectional shape composed of several layers of the three-dimensional shape. Also, the three-dimensional shape of implants and artificial bones can be obtained, for example, in the following way. In the first place, in order to obtain implants or artificial bones which fill defective sites, information on three-dimensional shape of the object may be obtained by computer simulating the shape of the bone of the object site so that the bones become in contrast. This is because a defective site generally has a counterpart whose shape is nearly in contrast with the defective site (for example, a right foot bone and a left hoot bone). Also, there are cases, such as manufacturing dental implants, that the shape of a diseased part itself is not suitable for duplicating. In this case, the shape of the object is drawn by 3DCG (three-dimensional computer graphics) based on the shapes of surrounding teeth and bones, and a computer obtains the information on the three-dimensional shape by inputting the 3DCG information, then information on each cross-sectional shape may be obtained by the computer based on the three-dimensional shape. In particular, when a signal from a pointing device is inputted in a CPU, the CPU reads out controlling program stored in the memory part such as CD-ROM or a hard disc based on the inputted signal. And the CPU scans X-ray figure stored in the memory part based on a direction from the controlling program, and a figure related to the three-dimensional shape is obtained by gathering a plurality of scanned two-dimensional figures. Note that since the X-ray photograph of bone or tooth part, and that of flesh part (or nervous part) are different in contrasting density, when the figure is obtained by scanning the X-ray photograph, an outline may be obtained from parts largely different in contrasting density. Also, patterning information of the bone part and the flesh part may be stored by obtaining the information, which is obtained by evaluating whether the contrasting density of the parts surrounded by the outline is in the range of predetermined value, or by comparing contrasting density of the part surrounded by the outline. Furthermore, when a figure concerning three-dimensional shape is obtained, the three-dimensional shape is, for example, sliced in the direction of Z-axis (the direction from the earth to the air), thereby obtaining cross sectional shape of each of a plurality of layers.

When contour lines are drawn on the surface of a bone model, contour lines corresponding to each height should be drawn by analyzing the height based on the shape of an obtained object (the shape of a bone model to be produced) by well-known methods. The rapid prototype method is to produce three-dimensional figures by laminating pluralities of layers. So, when each layer is produced, the top part and the bottom part of each layer may be colored. Also, for example, it is preferred to provide a colored layer every 2 to 100 layers. In this way, a bone model whereon contour lines are drawn every proper height can be obtained. Concretely, a predetermined part of each layer is colored with a coloring agent or an ink. It may also produce a colored layer by producing a bone model based on a program which is to form layers whereto a coloring agent and the like is added every predetermined layer.

When contour lines are drawn on the surface of a bone model, for example, a program in which, an obtained bone model is set so that (the front view of) the three dimensional data of the bone model is shown on the two-dimensional monitor, and grid are pasted to the two dimensions data, then the grid are drawn on the surface thereof, is preferred to be used.

The thickness of the layers may be adjusted as appropriate according to input information from the pointing device and the like. It may also be controlled according to a preset value. If the thickness of the layer is too thick, an elaborate hardening body cannot be obtained, and there is a problem that the hardness for maintaining the shape cannot be achieved by adding water drops thereto by using devices such as printing mechanism. On the other hand, if the thickness of the layer is too thin, too many cross-sectional figures must be obtained, thereby causing a burden on the computer hardware resource, and too much time is required for forming a figure. From this perspective, the thickness of each layer is, for example, 1x10 µm to 5 mm. It may be 1x10 µm to 5 mm, or may be 1x10² µm to 1 mm. Note that the thickness of each layer is preferred to be uniform, but may not be uniform.

The first cross-sectional figure forming step (step A2-1) comprises the steps of: reading out information on the cross-sectional shape of a first layer from the information on the cross-sectional shape obtained in the cross-sectional shape obtaining step; and forming the first cross-sectional figure duplicating the cross-sectional shape by using a figure-forming composition based on the read out information.

The second cross-sectional figure forming step (step A2-2) comprises the steps of: reading out information on the cross-sectional shape of a second layer from the information on the cross-sectional shape obtained in the cross-sectional shape obtaining step, the second layer being located on the upper layer of the first cross-sectional figure; and forming the second cross-sectional figure so as to be overlapped with the first cross-sectional figure, the second cross-sectional figure duplicating the cross-sectional shape from a figure-forming composition based on the read out information.

Next, wherein the three-dimensional figure obtaining step repeats, an upper layer cross-sectional figure forming step for forming an upper layer cross-sectional figure, in the same way as the second cross-sectional figure forming step, reading out information on the cross-sectional shape of the layer to be formed from the information on the cross-sectional shape obtained in the cross-sectional shape obtaining step; and forming the cross-sectional figure of the layer so as to be overlapped with the cross-sectional figure obtained in the former cross-sectional figure forming step, the cross-sectional figure of the layer duplicating the cross-sectional shape from a figure-forming composition based on the read out information.

A method for forming a three-dimensional figure duplicating a shape of an object, wherein at least one or more of the cross-sectional figure forming steps comprise: a figure-forming composition layer obtaining step for forming figure-forming composition layers by stratifying powders of the figure-forming composition above described; and a water adding step for moistening a predetermined part of the figure-forming composition layer by adding water to the figure-forming composition layer based on information on the cross-sectional shape of the layer, the layer formed in the figure-forming composition layer obtaining step.

Hereinafter, examples of each cross-sectional figure forming step is explained. In each cross-sectional figure forming step, the CPU receive a direction from the controlling program, and read out information on the thickness of a figure-forming composition layer, then outputs the information from the input/output device. The three-dimensional figure forming part having received the information on the thickness shifts the movable table downward following an order from the computer. The downward shift distance corresponds to the thickness of the figure-forming composition layer. The information on the downward shift distance is also outputted from the computer. And the movable table moves based on the shift distance information. Note that if the thickness of each layer is the same, the memory part of the three-dimensional figure forming part stores this information, and may use the same information in forming each layer.

Next, the CPU receives the direction from the controlling program, and, for example, reads out information on the thickness of the figure-forming composition layer, computes the amount of figure-forming composition suitable for forming the figure-forming composition layer, then outputs the information of the amount from the input/output part. This amount may be fixed, and having transmitted to the three-dimensional figure forming part, it may be stored in the store part of the three-dimensional figure forming part, and the same information may be used for forming each layer. The three-dimensional figure forming part which have received information on this figure-forming composition layer, based on the direction from the computer, makes the figure-forming composition layer forming part take out figure-forming composition powder from the figure-forming composition powder storing part, then releases the powder on the table. The three-dimensional figure forming part may control the figure-forming composition layer forming part to uniform the figure-forming composition layer by moving a squeegee or a spatula. In this way, the figure-forming composition layer is formed on the movable table (if already a layer is formed, another layer will be formed on a figure-forming composition layer formerly formed).

Next, having received a direction from the controlling program, the CPU reads out information on the cross-sectional shape of each layer or information on pattering, and outputs the information from the input/output part. The three-dimensional figure forming part, based on a direction from the computer, activates a printing part, and adds water or predetermined aqueous solution (water, cross-linker solution, binder aqueous solution for rapid prototyping) to the figure-forming composition layer. This mechanism can be easily achieved by using a controlling mechanism of a well known printer. Note that the conditions such as the composition, the density, and the amount of water or aqueous solution to be added can be adjusted as appropriate. For example, information on these conditions is inputted from the pointing device, and the inputted information is stored in the store part of the computer. Based on the information on these conditions, the CPU reads out necessary information and makes the computing part to perform computing, and controls the operation of the printing part. The printing part uses ordinary printing techniques except adding water instead of ink. The liquid binder material added to the figure-forming composition layer may be organic or inorganic. Typical organic binder material used is a ceramic precursor such as polymer resin or polycarbosilazane. Inorganic binder is used when a binder is mixed with the final material, in which silica is generally used.

Ordinarily, in the step of forming each layer, the amount of water which is more than the amount necessary for accelerating hydration reaction is repeatedly added and dried. But, in the method for forming three-dimensional figure of the present invention (the method for forming hardening material of the present invention), the hydration reaction of gypsum is not need to be completed in the above step. So, in each cross sectional figure-forming step, for example, when the amount of water necessary for hydrating the figure-forming composition completely is assumed to be 100 weight parts, the amount of water to be added may be, for example, 1 to 50 weight parts, 1 to 20 weight parts, 2 to 10 weight parts, or 3 to 5 weight parts. This little amount of water is not sufficient for completing the hydration reaction of gypsum. However, in the present invention, with this little amount of water, layers with hardness which is enough for maintaining least hardness can be obtained rapidly. Also, since the amount of water is little, the water can be prevented from spreading to unintended part, thereby obtaining layers having desired cross-sectional structures. In particular, in case of obtaining a cross-sectional structure which has more than two kinds of patterning, it is necessary for preventing two kinds of water or aqueous solution from being mixed. With little amount of water to be added, these two kinds of water or aqueous solution can be effectively prevented from being mixed.

Having repeatedly performed the cross-sectional figure forming step, it is preferred to dry the resultant layered product until it has a certain level of hardness. The drying may be performed in low humidity high temperature atmosphere (for example humidity 0 to 10%, temperature 50 to 2x10² degrees Celsius), but may be performed at ordinary temperatures and pressures. The drying time at an ordinary temperature and pressure is preferred to be adjusted as appropriate, according to the size, the moisture percentage, and the thickness of each layer of the resultant three-dimensional figure. The examples of the drying time include 1 minute to 1 hour, 5 minutes to 3x10 minutes, and 5 minutes to 2x10 minutes. Namely, in using the rapid prototype process in the present invention, figure-forming composition containing a large amount of polyvinyl alcohol resin is used, so a figure-forming composition having relatively high level of hardness could be obtained. And in this step, the figure-forming composition need not contain enough water, so drying time can be remarkably shortened. Then, after drying the layered product, a three-dimensional figure duplicating the shape of an object can be obtained. Note that, the drying is preferred to be performed in a deaeration step, for example deaeration by decompression, because in the deaeration step, the drying can be performed quite rapidly.

It is highly likely that the three-dimensional figure obtained in the above way contains gypsum whose hydration reaction does not proceeded. So, the hardness thereof is assumed to be low compared to that of three-dimensional figure whose hydration reaction is preceded. However, by patterning layers with a little amount of water, water can be prevented from spreading to unintended parts, thereby preventing the unintended parts from being hardened. So, this method for forming three-dimensional figure is useful for forming three-dimensional figure having a sophisticated shape in a short period. On the other hand, the three-dimensional figure obtained in the above way has a sophisticated shape, but it is assumed that the hardness thereof is low because the hydration reaction is not sufficiently preceded. In order to obtain enough hardness, it is preferred that hydration reaction be proceeded according to the method for forming hardening object described later.

As above described, the method for producing a bone model is the method for obtaining hardening body having enough hardness basically by the following procedures. The resultant three-dimensional figure obtained in the above each step is soaked into water or aqueous solution, thereby accelerating hydration of gypsum. And then the resultant figure is dried.

Namely, the method for producing a bone model relates to the method comprising: a gypsum powder removing step (step B1) for removing unconsolidated figure-forming composition powder from the three-dimensional figure basically obtained by one of the method for forming a three-dimensional figure above described; a water adding step (step B2) for adding water to the three-dimensional figure whose unconsolidated powders were removed in the gypsum powder removing step; and a drying step (step B3) for drying the three-dimensional figure to which water was added in the water adding step. Hereinafter, each step is explained.

Gypsum powder removing step (step B1) is a step for removing powders of unhardened figure-forming composition from the three-dimensional figure. In this step, for example, unhardened gypsum powders are blown off by an airbrush. The amount of airflow, the shape of the airbrush, and the like may be adjusted as appropriate, and a well known airbrush can be used. The time required for the gypsum powder removing step is also adjusted as appropriate. The specific example is 5 minutes to 1 hour, and 10 minutes to 30 minutes is preferred.

Water adding step (step B2) is a step for adding water to the three-dimensional figure whose powders are removed in the gypsum powder removing step. In this water adding step, enough water for accelerating hydration reaction of gypsum is preferred to be added to a three-dimensional figure. In this water adding step, three-dimensional figure is soaked in water or predetermined aqueous solution. In this process, since unnecessary powders are removed in the former gypsum powder removing step, figure-forming composition powders unnecessary for forming the shape of the three-dimensional figure can be prevented from sticking to the three-dimensional figure.

A preferred embodiment of the method for forming three-dimensional structures according to the third aspect of the present invention includes the water adding step (step B2) which comprises: an atomizing step (step B2-1) for attaching water on the surface of the three-dimensional figure by misting water or by exposing the three-dimensional figure to high humidity atmosphere, the three-dimensional figure being removed unconsolidated powders therefrom in the gypsum powder removing step; and a soaking step (step B2-2) for soaking the three-dimensional figure in water after the atomizing step.

There is a problem that the shape of three-dimensional figure is deformed if the resultant three-dimensional figure is soaked in water immediately. Considering this problem, the following procedures are taken in this embodiment of the method for forming three-dimensional structure. Firstly, water is added on the surface (preferably on all over the surface) of the resultant three-dimensional figure, thereby accelerating the hardening reaction, at least, on the surface thereof through hydration reaction of gypsum (preferably followed by drying the figure) and preventing the figure from being deformed. Then, the hardening reaction is further accelerated by soaking the figure in water. In the atomizing step, for example, water or predetermined aqueous solution (preferably water, cross-linker solution, or binder aqueous solution) is sprayed on the surface of the three-dimensional figure by using a known spray. Or water is added on the surface of the three-dimensional figure by placing the three-dimensional figure in high humidity atmosphere. Then, having sprayed water, the figure is dried, and then soaked in water. The figure may be dried in low humidity high temperature atmosphere (for example humidity 0 to 10 percent, temperature 50 to 2x10² degrees Celsius), but may be dried at an ordinary temperature and pressure. The drying time at an ordinary temperature and pressure is preferred to be adjusted according to the size, the moisture percentage, and the thickness of each layer of the resultant three-dimensional figure, as appropriate. The examples of the drying time include 1x10 minutes to 2 hours, 15 minutes to 1 hour, and 2x10 minutes to 4x10 minutes. In the soaking step, the three-dimensional figure is soaked in sufficient water or aqueous solution. The soaking time may be adjusted as appropriate according to the size of the three-dimensional figure. The examples of the soaking time include 1x10 minutes to 2 hours, 15 minutes to 1 hour, and 2x10 minutes to 4x10 minutes.

A preferred embodiment of the method for producing a bone model is the above described method for producing a bone model comprising the water adding step (step B2) which comprises: (1) an atomizing step for attaching water on the surface of the three-dimensional figure by misting water or by exposing the three-dimensional figure to high humidity atmosphere, the three-dimensional figure being removed unconsolidated powders therefrom in the gypsum powder removing step, and a soaking step for soaking the three-dimensional figure in cross-linker solution after the atomizing step; (2) an atomizing step for attaching cross-linker solution on the surface of the three-dimensional figure by misting cross-linker solution or by exposing the three-dimensional figure to high humidity atmosphere of cross-linker solution, the three-dimensional figure being removed unconsolidated powders therefrom in the gypsum powder removing step, and a soaking step for soaking the three-dimensional figure in cross-linker solution after the atomizing step; or (3) an atomizing step for attaching water on the surface of the three-dimensional figure by misting water or by exposing the three-dimensional figure to high humidity atmosphere, the three-dimensional figure being removed unconsolidated powders therefrom in the gypsum powder removing step, and a soaking step for soaking the three-dimensional figure in water and then in cross-linker solution after the atomizing step. In particular, step (1) or (3) is preferred to be used for figure-forming composition containing acetoacetyl group modified polyvinyl alcohol resin. This is because, in view of hardness or uniformity of the three-dimensional structure, it is preferred to promote bridging reaction with a cross-linker, after having developed chelate structures with water.

In this way, by adding a cross-linker such as cross-linker solution, cross-linking reaction proceeds in the three-dimensional figure, and a three-dimensional structure having enough hardness can be obtained. The atomizing step and the soaking step are performed in the same way as above explained. The density of cross-linker solution is adjusted as appropriate according to the kind of polyvinyl alcohol resin used and the hardness of the hardening body to be obtained. The concentration of cross-linker solution is specifically 1x10⁻² to 2x10 volume percent, preferably 1x10⁻¹ to 1.5x10 volume percent. As cross-linker solution, in place of or together with amine cross-linker solution such as ethylenediamine or diethanolamine, the following materials can be used as appropriate: aldehyde compound such as formaldehyde or glyoxal; methylol compound such as melamine-formaldehyde condensate or urea-formaldehyde condensate; boron-containing compound such as boracic acid or borax; isocyanate compound such as 2,4-tolylene diisocyanate, 2,6-tolylene diisocyanate, m-phenylene diisocyanate, p-phenylene diisocyanate or 4,4-diphenylmethane diisocyanate; or silane coupling agent. Among them, as a cross-linker, amine cross-linker solution such as ethylenediamine or diethanolamine is preferred. In particular, as demonstrated in the example described later, one or both of ethylenediamine and diethanolamine is more preferred.

The drying step (step B3) is a step for drying the three-dimensional figure which is added water in the water adding step. The drying may be performed in low humidity high temperature atmosphere (for example, humidity 0 to 10%, temperature 50 to 2x10² degrees Celsius), but may be performed at an ordinary temperature and pressure. The drying time at an ordinary temperature and pressure is preferred to be adjusted according to the size, the moisture percentage, and the thickness of each layer of the resultant three-dimensional figure, as appropriate. The examples of the drying time include 1 hour to 4 days, 4 hours to 3 days, and 6 hours to 2 days.

In the present invention, however, the drying time is preferred to be from 1 hour to 4 hours, because the bone model is preferred to be produced relatively quickly.

### Figure-forming Material Filling Step (Step 2)

The figure-forming material filling step is a step for filling figure-forming material in a bone deficit site of the bone model obtained in the bone model producing step. A figure of a bone filling material can be obtained by filling figure-forming material in the bone deficit site.

Figure forming material is not specifically limited if a figure of a bone filling base material is obtained from a well known figure-forming material as appropriate. But the figure forming material is preferably distinguished from a bone model. Specifically, figure forming material which differs from the bone model in one of the X-ray permeability, the infrared ray permeability, or the ultraviolet ray permeability is preferred. For example, the X-ray permeability, the infrared rays permeability, or the ultraviolet rays permeability of the figure of a bone filling base material whereon figure forming material is combined are equal to or less than 90 percent or equal to or more than 110 percent of those of a bone model. If the figure-forming material has the above X-ray permeability, infrared ray permeability, or ultraviolet ray permeability, a figure of a bone filling base material whereon figure forming material is combined can be easily distinguished from a bone model. The figure-forming material, for example, is made from raw material which is the same as that of a bone model including coloring agent, metallic powder, or metallic oxide powder

The figure-forming material, for example, contains wax ingredient (e. g., dental wax) as the main component. The examples of the wax ingredient, other than paraffin wax, include one or a mixture of: beeswax; microcrystalline wax; dammar; rosin; candelilla wax ; carnauba wax; or montan wax. Among them, the one which mainly contains paraffin wax is preferred. The examples of the wax component of the figure-forming material containing paraffin wax as the main component include: wax component having flexibility and adhesiveness by adding beewax or microcrystalline wax thereto; wax component having improved strength and hardness as well as having adhesiveness by adding dammar or rosin thereto; and wax component having glazing surface by adding carnauba wax which has high-melting point thereto. Another example is a synthetic resin which is a mixture of hydrocarbon resin and ethylene-vinyl copolymer resin. In particular, ethylene-vinyl copolymer resin is mixed in paraffin wax so that the ethylene-vinyl copolymer resin is preferably 1 to 5 weight parts when the total weight is 100 weight parts. More specific examples of dental wax include paraffin wax, rolling wax, proline wax, corben wax, pro-utility wax, bite rim stick or carving wax. Number average molecular weight (Mn) measured by a gel permeation chromatography (GPC) is from 400 to 5,000, preferably from 800 to 5,000, more preferably from 1,000 to 3,000, further preferably from 1,500 to 2,500. The ratio between the weight-average molecular weight measured by a GPC and the number average molecular weight (Mw/Mn) of the wax is equal to or less than 4.0, preferably equal to or less than 3.5, more preferably equal to or less than 3.0. Note that weight average molecular weight (Mw) and the number average molecular weight (Mn) are polystyrene equivalent measured by gel permeation chromatography (GPC). The GPC measuring is performed under the following condition: temperature 140 degrees Celsius; solvent ortho-dichlorobenzene.

As for wax, other than generally used red dental wax, white wax, yellow wax, black wax, or the like can be used as appropriate. White wax is produced by mixing the titanium oxide, which is a white pigment, in wax such as the paraffin wax. Yellow wax, for example, is produced by mixing titan yellow, which is a kind of the titanium oxide (yellow pigment), in wax such as paraffin wax. Black wax, for example, is produced by mixing aniline black, which is a black pigment, in wax such as paraffin wax.

The figure-forming material is preferred to include inorganic powder, organic powder, surfactant metal salt powder, pigment, coloring agent, metallic powder, metallic oxide powder, or the like, so that a figure of a bone filling base material whereon figure-forming material is combined can be distinguished from a bone model in one of the X-ray permeability, the infrared ray permeability, or the ultraviolet ray permeability.

Specific examples of inorganic powder include titanium oxide, zirconium oxide, zinc oxide, cerium oxide, magnesium oxide, barium sulfate, calcium sulfate, magnesium sulfate, calcium carbonate, magnesium carbonate, talc, mica, kaolin, sericite, muscovite, synthetic mica, phlogopite, lepidolite, biotite, lithia mica, silica acid, silicic anhydride, aluminium silicate, magnesium silicate, magnesium aluminium silicate, calcium silicate, barium silicate, strontium silicate, tungstic acid metal salt, hydroxyapatitte, vermiculite, higilite, bentonite, montmorillonite, hectorite, zeolite, ceramics powder, calcium secondary phosphate, alumina, aluminum hydroxide, boron nitride, and silica.

Specific examples of inorganic powder include polyamide powder, polyester powder, polyethylene powder, polypropylene powder, polystyrene powder, polyurethane, benzoguanamine powder, polymethyl benzoguanamine powder, tetrafluoroethylene powder, polymethyl methacrylate powder, cellulose, silk powder, nylon powder, 12 nylon, 6 nylon, crosslinkable silicone impalpable powder having a cross-linked structure of dimethyl silicone, impalpable powder of polymethyl silsesquioxane, styrene acrylic acid copolymer, divinylbenzene styrene copolymer, vinyl resin, urea resin, phenol resin, fluororesin, silicone resin, acrylic resin, melamine resin, epoxy resin, polycarbonate resin, mycrocrystallite fiber powder, starch powder, lauroyl lysine. Further, organic powder most of which is composed of - [Si-O-] ₙ- frame can be used. In this case, the molecule may have a -Si (CH₂CH₂) ₘ-Si- coupling therein.

Specific examples of surfactant metal salt powder (metal soap) include zinc stearate, aluminum stearate, calcium stearate, magnesium stearate, zinc myristate, magnesium myristate, zinc cetyl phosphate, calcium cetyl phosphate, Sodium zinc cetyl phosphate.

Specific examples of colored pigment include: inorganic red pigment such as iron oxide, iron hydroxide, and iron titanate; inorganic brown-based pigment such as the γ - iron oxide; inorganic yellow-based pigment such as yellow iron oxide, and yellow ocher; inorganic black pigment such as black iron oxide, and carbon black; inorganic purple pigment such as manganese violet, and cobalt violet; inorganic green pigment such as chromium hydroxide, chromium oxide, cobalt oxide, and cobalt titanate; inorganic blue-based pigment such as iron blue, and ultramarine blue; laked tar-based pigment; laked natural pigment; and synthetic resin powder which combined powders thereof.

Specific examples of pearl pigment include titanium oxide coating mica, bismuth oxychloride, titanium oxide coating bismuth oxychloride, titanium oxide coating talc, fish scale guanine, titanium oxide coating coloration mica.

Specific examples of metallic powder pigment include aluminum powder, cupper powder, and stainless powder.

Specific examples of natural pigment are powder of carminic acid, laccaic acid, carthamin, brazilin, and crocin. These powders can be combined or can be processed with general oil, silicone oil, a fluorine compound, or surfactant as long as it maintains the effect of the present invention. Organohydrogen polysiloxane having reactiveness, organopolysiloxane having a hydrolysable alkoxysilane group, and acryl silicon-based copolymer having a hydrolsable silyl group can also be used. One or more than one kind of them can be mixed as needed. A specific example of the pigment is the above titanium oxide. Metallic powder or the powder of metallic oxide is not specifically limited, and a well-known metallic powder or the powder of metallic oxide can be used as appropriate. The preferred example of the metallic powder or the powder of metallic oxide is titanium oxide, more preferably titanium oxide of anatase type or rutile type. The particle size of the metallic powder or the powder of metallic oxide (analyzed based on JISR1619) is from 1x10²nm to 5x10³nm, preferably form 5x10²nm to 3x10³nm. As demonstrated in the example below, figure-forming material, for example, contains wax of more than 90 weight percent (preferably more than 95 weight percent) of the total amount, and contains the metallic powder or the powder of metallic oxide of 2 to 5 weight percent of the total amount, more preferably contains the metallic powder or the powder of metallic oxide of 3 to 4 weight percent of the total amount.

Since a bone model is formed based on the bone of a patient by the PR method and the like, surgeons and the like can fill figure-forming material into the bone deficit site of the bone model. In this way, since a practitioner oneself forms a figure of bone filling base material on the precisely formed bone model, a bone filling base material which is usable for a practitioner can be obtained. In particular, since the figure-forming material, for example, contains paraffin wax and the like as the main component, it easily becomes flexible in hot water. So, the figure-forming material having the flexibility is preferred to be filled in a bone deficit site. Also, when the figure-forming material is filled in a bone deficit site of a bone model, bone filling base material may be automatically filled in the deficit site of the bone model.

The figure-forming material filled in a deficit site of a bone model, for example, contains paraffin wax as the main component. So, it will be hardened to be a figure of a bone filling base material by leaving it as it is. This figure of a bone filling base material is used for treatment of a patient. And it precisely fits in the shape of a bone deficit site.

### Bone Filling Base Material Producing Step (Step 3)

The bone filling base material producing step is a step for producing a bone tilling base material which is filled in the bone deficit site based on the figure-forming material (or a figure of a bone filling base material) filled in the bone deficit site of the bone model in the figure-forming material filling step. Figure-forming material is filled in the bone deficit site of the bone model in the figure-forming material filling step, and the figure of the bone filling base material whereon figure-forming material is combined is distinguished from the bone model in some way. So, a bone filling base material is preferred to be produced by using this figure of bone filling base material. Also, a bone filling base material may be produced by the powder injection molding method or its modified method, by using a mold which is produced based on the shape of the obtained figure of a bone filling base material.

In particular, a bone filling base material is produced in the following way. Since a figure of a bone filling base material and a bone model has different X-ray permeability or reflectivity, each bone model containing a figure of a bone filling base material is photographed by an X-ray CT and the like. The obtained X-ray permeability or reflectivity is sent to a control equipment such as a personal computer. The control equipment grasps the shape of the figure of the bone filling base material based on the difference of an X-ray permeability or reflectivity, sends the information of the shape of the bone filling base material to a production unit of a bone filling base material, and outputs a direction to the production unit for producing a bone filling base material based on the shape of the bone filling base material. The production unit is, for example, a production unit based on the PR method, and a bone filling base material is produced by the PR method or the above explained method. Note that when a bone filling base material is produced by the PR method, compound powders (such as calcium phosphate-based material) which is explained below is preferred to be used as raw material powders as appropriate.

Having produced a bone filling base material, a blocking agent as well as a pharmaceutical agent is preferred to be impregnated into or pasted to the base material. Of course, the blocking agent and the pharmaceutical agent may be impregnated into or pasted to the base material at the same time or separately. The size of the bone filling material above explained was relatively small. Although such small size of a bone filling material can be produced according to the above explanation, the bone filling material basically is to be embedded into a bone defect site. So, the time for impregnating the blocking agent or the pharmaceutical agent is preferred to be extended by 1.5 to 3 times that of the above explanation. And the concentration of the blocking agent or the pharmaceutical agent is preferred to be increased as appropriate.

The other preferred aspect of the present invention is a drug release controlling carrier comprising a calcium-based material, and a blocking agent of the calcium-based material. The release controlling carrier, which can control drug release, is administered in vivo together with a pharmaceutical agent. Since the carrier is basically the same material as the above described bone filling material, the above described composition is adopted as appropriate, and the carrier can be produced in the same way as above described. The drug release controlling carrier containing a pharmaceutical agent is administered into a certain diseased part of a patient by cutting the part in the same way as the bone filling material above described. Namely, the present invention provides a method for treating certain diseases, the method including a step for administering the release controlling carrier to an object (e.g., a human patient). The present invention also provides a usage of the release controlling carrier and a certain pharmaceutical agent for producing a therapeutic agent of a certain disease.

### EXAMPLE 1

### Drug Release Control

In Example 1, an experiment was made to demonstrate that the bone filling material of the present invention could control drug release. The reagents used in Example 1 were an α-TCP made by Taihei Chemical Industrial Co., Ltd., a sodium chondroitin sulfate CS made by Seikagaku Kogyo Co., Ltd., a disodium succinate made by Wako Pure Medicine Co., Ltd., injection solvate made by Otsuka Pharmaceutical Co., Ltd., an L-glutamine made by Nacalai Tesque Co., Ltd., an L-serine made by Nacalai Tesque Co., Ltd., a dextran made by Meito Sangyo Co., Ltd., and a trehalose made by Hayashibara Institute for Chemical Research Co., Ltd.

A Z printer 406 made by Z corporation was used to produce a bone filling material. "LVDV" which is a digital viscometer made by Brookfield Co., Ltd. was used to measure viscosity. A UL (ultra low viscosity) adapter was used when the viscosity was measured. Bending strength was measured by a rheometer made by Sun Science Co., Ltd.

### 1-1.Method for Producing Base Material

Test pieces were produced from α-TCP as a main raw material by the Z printer 406. One of the test pieces was columnar-shaped material with 5 mm of diameter and 1.5 mm of height, and the other test piece was a square-columnar-shaped material of 3 mm x 4 mm x 36 mm. Injection sclerosing solution was sprayed to these test pieces. The sclerosing solution was composed of 4 weight percent CS, 12 weight percent disodium succinate, and 84 weight percent injection solvate. It is estimated that the amount of CS can be adjusted as appropriate, for example, in the range from 1 to 12 weight percent. After having sprayed the injection sclerosing solution to the test pieces, they were moistened for 24 hours at 35 degrees Celsius. The moistening step is to be continued for from 12 hours to 2days. The test pieces were dried for 24 hours in normal temperature, under negative pressure, and base materials were obtained. They may also be dried in high temperature, under low humidity environment.

### 1-2.Step for Pasting Blocking Agent

Next, the base material obtained in the above step is soaked in a solution of the blocking agent, and the material was left as it was for 3 hours at normal temperature, thereby impregnating the blocking agent into the base material. The blocking agent may be pasted to the base material by a well known method. After the blocking agent was impregnated into the base material, the material was pre-dried and sterilized in an autoclave for 20 minutes at 121°C. And then, the base material was dried for 24 hours at normal temperature and in a low humidity environment. In this way, test pieces were produced. The base material may also be dried at high temperature and in a low humidity environment. For the purpose of comparison, a base material to which injection sclerosing solution was not impregnated or pasted, and a base material to which only injection solvate was impregnated or pasted were prepared. The types and the weight concentration of the blocking agent used in this step are shown in table 1.

**TABLE 1 Composition of Blocking Solution**

| Test piece | Blocking Agent | Solvent Medium | Concentration [%] |
|---|---|---|---|
| a | L-glutamine | Injection Solvate | 5 |
| b | L-serine | | 5 |
| c | | | 21 |
| d | Dextran 40 | | 1 |
| e | | | 13 |
| f | Trehalose | | 5 |
| g | | | 37.9 |
| h | Injection Solvate | - | - |
| i | none | - | - |

### 1-3.Step for Pasting Pharmaceutical Agent

Next, FGF as a pharmaceutical agent was pasted to the test pieces obtained in the above step. In this way, bone filling materials were obtained.

### 1-4.Bending Strength Measurement

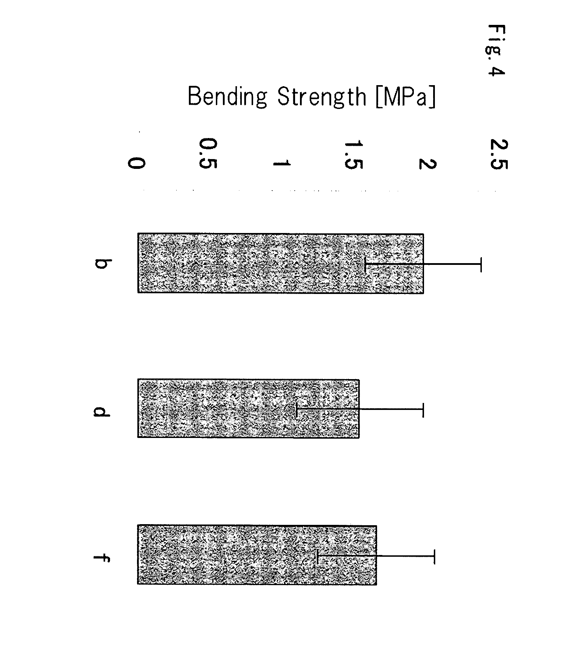

Bending strength measuring tests were performed on the square columnar shaped test pieces obtained in the above step. The results are shown in Fig. 4. Fig. 4 is a graph showing results of bending strength tests of test pieces. As is shown in Fig. 4, a bone filling material containing serine was the hardest of all.

### 1-5. Viscosity Measurement

Viscosity of blocking agents were measured by "LVDV" which is a digital viscometer made by Brookfield Co., Ltd. The results are shown in Table 2. It is noted that viscosity of L-glutamine could not be measured because it cannot be dissolved in water.

**Table 2 Results of Viscosity Measuring Tests of Blocking Agent**

| Test Piece | Blocking Agent | Solvent | Concentration [%] | Viscosity [mPa•s] | Torque [%] |
|---|---|---|---|---|---|
| B | L-serine | Injection Solvate | 5 | 1.31 | 10.9 |
| C | | | 21 | 2.04 | 17.0 |
| D | Dextran 40 | | 1 | 1.27 | 10.6 |
| e | | | 13 | 6.95 | 57.9 |
| f | Trehalose | | 5 | 1.27 | 10.6 |
| g | | | 37.9 | 4.55 | 37.9 |

It can be seen from Table 2 that the blocking agent containing 13 weight percent dextran exhibits excellent blocking agent properties because it has high viscosity and large torque.

### EXAMPLE 2

### In Vitro Release Evaluation

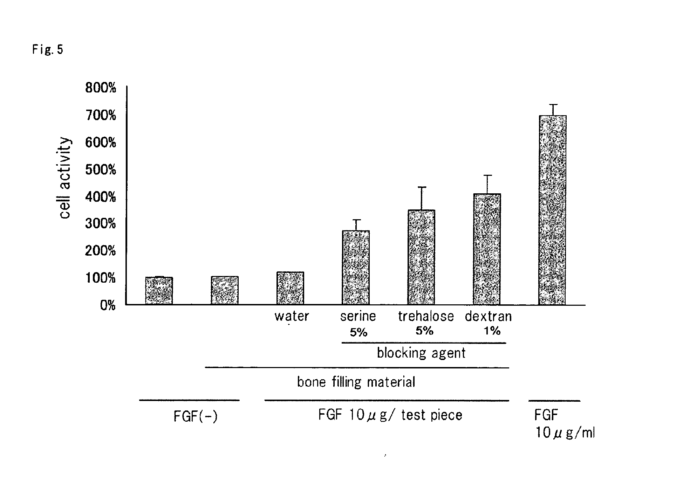

In vitro drug release of the bone filling material of the present invention is evaluated in the following way. 100 µL of MC3T3-E1 cells suspension, which are mouse-derived osteoblasts-like cells, were seeded onto 96 well plate at 5000 cells per well. They were pre-cultured for 24 hours in a CO₂ incubator. And then, 10 µg of FGF was pasted to each test piece, which was produced in Example 1, by dropping the FGF above the test piece. As a positive control, 10 µg/mL of FGF was directly added to the culture well. It was cultured for 48 hours in a CO₂ incubator. And then, 10µL of Cell Counting Kit-8 solution made by Dojin Chemistry Laboratories was added in each well, and was subjected to color reaction in a CO₂ incubator for 4 hours. The absorbance at 450nm was measured by a microplate reader. The results are shown in Fig. 5. Fig 5 is a graph showing absorbance to evaluate drug release in an in vitro experiment. It can be seen from Fig. 5 that the test pieces containing blocking agent are superior to the test pieces without containing blocking agent in FGF releasing ability. It can be seen, in particular, that Dextran is superior in releasing FGF.

### EXAMPLE 3

### In Vivo Bone Reproduction Ability Evaluation

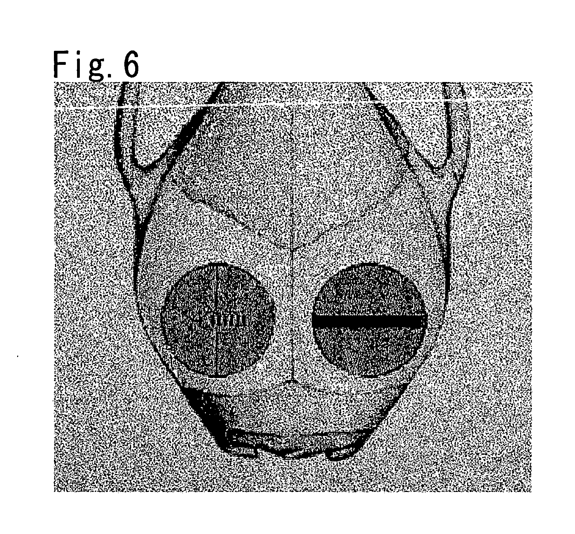

A circular all layer bone defective part, diameter of 4 mm, was made in a mouse skull by using a sterilizing disposable trepan. Fig. 6 is a photograph, in place of a drawing, showing a mouse skull whereon circular all layer bone defect part was formed. After having embedded each kind of bone filling material produced in Example 1 into the circular all layer bone defect part, the part was sutured up. After 4 weeks, the mouse was euthanazed and the skull was taken out. The skull was dyed with hematoxylin eosin (HE), and the X-ray was taken. Fig. 7 is an X-ray photograph, in place of a drawing, showing a cross-sectional surface of the skull. Fig. 7(a) shows a skull whose circular all layer bone defect part was filled with a bone filling material containing 5% of serine as a blocking agent. Fig. 7(b) is a partial enlarged view of Fig. 7(a). Fig. 7(c) shows a skull whose circular all layer bone defect part was filled with a bone filling material containing 5% of trehalose as a blocking agent. Fig. 7(d) is a partial enlarged view of Fig. 7(c). Fig. 7(e) shows a skull whose circular all layer bone defect part was filled with a bone filling material containing 1% of dextran as a blocking agent. Fig. 7(f) is a partial enlarged view of Fig. 7(e). Fig. 7(g) shows a skull whose circular all layer bone defect part was filled with a bone filling material without containing a blocking agent. Fig. 7(h) is a partial enlarged view of Fig. 7(g).

It can be seen from Fig. 7(g) and Fig. 7(h) that when the bone filling material without containing blocking agent was embedded in the defect part, the bone filling material was exfoliated from the tissues thereof, and bone regeneration could not be observed. On the other hand, as is shown in Fig. 7(a), when the bone filling material containing 5% of serine as a blocking agent was embedded in the circular all layer bone defect part, regeneration of bone cells was observed in the bone defect part. And the bone filling material remained on the regenerated cells. As is shown in Fig. 7(b), the regenerated bone cells were preferable. As are shown in Fig. 7(c) and Fig. (e), when the bone filling material containing trehalose and dextran as blocking agents was embedded in the circular all layer bone defect part, regeneration of bone cells was also observed in the bone defect part. And the bone filling material remained on the regenerated cells.

It can be seen from the above explanation that the bone filling material containing a calcium-based material, a blocking agent of the calcium-based material, and a pharmaceutical agent has preferred drug release property compared to a conventional bone filling material merely containing a pharmaceutical agent. In fact, it has excellent bone regeneration ability.

### EXAMPLE 4

Mouse skeletal muscle-derived C2C12 cells were seeded onto 24 well plate at 2.5x10⁴ cells per well. The culture medium was 500µL respectively. A C2C12 cell is a myoblast, which differentiated into an osteoblast by BMP-2, and expresses ALP (alkaline phosphatase). The cells were cultured in a CO₂ incubator for 24 hours. And then, each test piece produced in example 1 was put into the culture well. Noted that 500ng of BMP was added to a bone filling material. And then, it was cultured in a CO₂ incubator for a week.

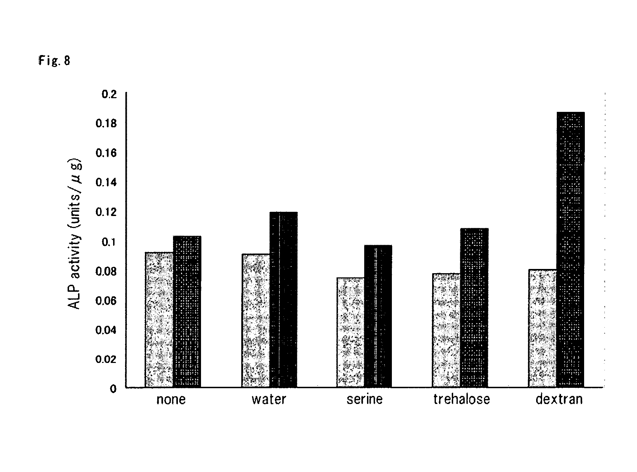

After the incubation, the effect of the BMP was measured by an ALP activity measurement kit. In particular, according to a manual of ALP activity measurement kit, it was subjected to color reaction at 37 degrees Celsius for 15 minutes. And then, the absorbance at 450nm was measured by a microplate reader. The results are shown in Fig. 8. Fig. 8 is a graph, in place of a diagram, showing a result of in vitro quantification test showing release control of BMP. The vertical axis shows ALP activity. And the horizontal axis shows, respectively, from left to right, a bone filling material without containing a blocking agent, a bone filling material and BMP without containing a blocking agent, a bone filling material capped with water, a bone filling material and BMP capped with water, a bone filling material capped with 5% serine, a bone filling material and BMP capped with 5% serine, a bone filling material capped with 5% trehalose, a bone filling material and BMP capped with 5% trehalose, a bone filling material capped with 1% dextran, a bone filling material and BMP capped with 1% dextran.

It can be seen from Fig. 8, that ALP activity was significantly enhanced by using dextran as a bone blocking agent compared to a case in which control and water were used as blocking agents. This means that the amount of BMP released from the bone filling material was increased. On the other hand, in the case in which serine or trehalose were used as blocking agents, ALP activity was not significantly enhanced. In the above example, release amount of FGF is increased by using them. Therefore, it can be seen that there exists a preferred blocking agent with respect to each bioactive substance. The above mentioned bone filling material can be also utilized as the carrier which controlled drug release property. Therefore it can be seen that the present invention can provide a preferred release controlling carrier of the drug.

### INDUSTRIAL APPLICABILITY

The bone filling material of the present invention is injected in a bone deformed part, a bone defect part, and an osteoporosis part, etc., or filled in a bone defect part, etc. It also used as a carrier for a certain pharmaceutical agent. So, the bone filling material and the release controlling carrier of the present invention can be used in the field of pharmaceutical industry.

## Claims

1. A bone filling material comprising:
a calcium-based material;
a blocking agent of the calcium-based material; and
a pharmaceutical agent.

2. The bone filling material according to claim 1,
wherein the calcium-based material is one or more kinds of hydroxyl apatite, carbonic acid apatite, fluorapatite, chlorapatite, β-TCP, α-TCP, calcium metaphosphate, tetra-calcium phosphate, octa-calcium phosphate, calcium hydrogen phosphate, calcium dihydrogen phosphate, calcium pyrophosphate, salts thereof, and solvates thereof.

3. The bone filling material according to claim 1,
wherein the calcium-based material is a compound of C₃-C₁₀ dicarboxylic acid and one or more kinds of chemical compounds selected from the group consisting of "hydroxyapatite, carbonate apatite, fluorapatite, chlorapatite, β-TCP, α-TCP, calcium metaphosphate, tetra-calcium phosphate, octa-calcium phosphate, calcium hydrogen phosphate, calcium dihydrogen phosphate, calcium pyrophosphate", salts thereof, or solvates thereof.

4. The bone filling material according to claim 1,
wherein the blocking agent comprises one or pluralities of groups selected from the group consisting of "carboxyl group, amino group, hydroxyl group, one of the groups represented by the equation (I) below, -NHR¹, -N(R¹)₂, -SH, -R¹, -NHCOR¹, -NHSO₂R¹, -NHCN, -CH₂(CN), -CH(CN)₂ and -SH, wherein the -R¹ represents C₁₋₆ alkylgroup", and
wherein the molecular weight (the number average molecular weight) of the blocking agent is from 75 to 1x10⁵.

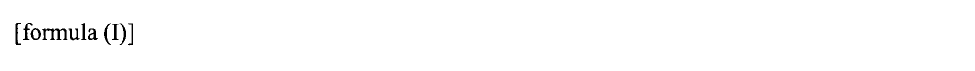

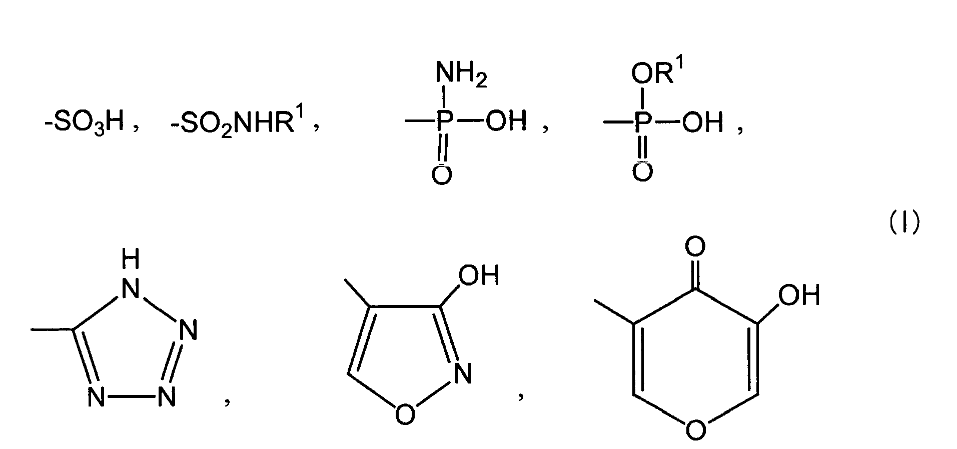

5. The bone filling material according to claim 1,
wherein the blocking agent is one or more kinds selected from an amino acid, a peptide, a protein, a saccharide, a glycoprotein, and a glycolipid.

6. The bone filling material according to claim 1,
wherein the blocking agent includes one or more kinds selected from amino acid, amino acid derivative, or a pharmaceutically acceptable salt thereof, the amino acid being selected from the group consisting of "alanine, arginine, asparagine, aspartic acid, cysteine, glutamine, glutamic acid, glycine, histidine, isoleucine, leucine, Lysine, methionine, phenylalanine, proline, serine, threonine, tryptophan, tyrosine, and valine",
wherein a carboxyl group may be replaced with one of the groups represented by the formula (I) (in the formula (I) and hereinafter, R¹ represents C₁₋₆ alkyl group),
wherein an amino group may be replaced with -NHR¹, - N(R¹)₂, -SH or -R¹, and
wherein a hydroxyl group may be replaced with -NHCOR¹, -NHSO₂R¹, -NHCN, -CH₂ (CN), -CH (CN)₂, or -SH.

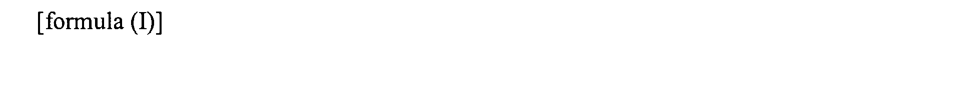

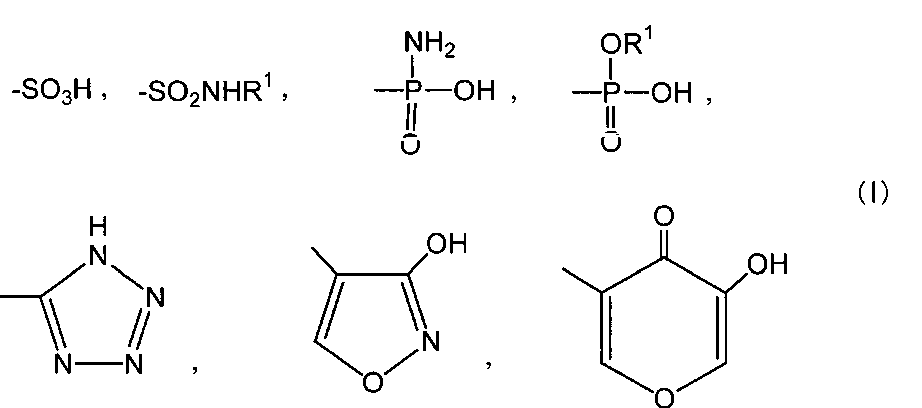

7. The bone filling material according to claim 1,
wherein the blocking agent includes one or more kinds selected from a saccharide, a saccharide derivative, or a pharmaceutically acceptable salt thereof, the saccharide being selected from the group consisting of "pentose, hexose, heptose, octose, nonose, decose, maltose, cellobiose, gentiobiose, melibiose, lactose, turanose, sophorose, trehalose, isotrehalose, sucrose, isosaccharose, maltotriose, cellotriose, hyaluronic acid, teichoic acid, colominic acid, chondroitin sulfate, dermatan sulfate, heparin, heparan sulfate, keratan sulfate, chitin saccharide, pullulan, guar gum, lambda carrageenan, traganth gum, pectin, mannan, dextran, and maltodextran", and
wherein a hydroxyl group may be replaced with -NHCOR¹, -NHSO₂R¹, -NHCN, -CH₂ (CN), -CH (CN)₂, or -SH (wherein R¹ represents C₁₋₆ alkyl group).

8. The bone filling material according to claim 1,
wherein the blocking agent is one or both of serine and dextran.

9. The bone filling material according to claim 1,
wherein, when the pharmaceutical agent is administered in a sufficient amount of water at normal temperature and under normal pressure, the release amount of the pharmaceutical agent within one hour from the administration becomes from 1.5 to 1x10² times in comparison with a case in which a bone filling material without containing the blocking agent is used.

10. The bone filling material according to claim 1,
wherein the pharmaceutical agent is one or more kinds of osteogenesis/chondrongenesis promoter (including chondrongenesis promoting factor), joint disease therapeutic agent, bone/cartilage disease preventive/therapeutic agent, bone regenerating agent, osteoclastic inhibitor, angiogenetic promoter, antibacterial agent, antibiotics, and anticancer agent.

11. The bone filling material according to claim 1, having pluralities of protrusions,
wherein a pharmaceutical agent is impregnated into or pasted on the surface of the protrusions.

12. The bone filling material according to claim 1, having a shape which is designed considering a bone shape of a patient, the bone shape being produced by the rapid prototyping method.

13. The bone filling material according to claim 1, being cementitious or gelatinous material.

14. The bone filling material according to claim 1,
wherein the calcium-based material is one or more kinds of hydroxyapatite, β-TCP, α-TCP, salts thereof, and solvates thereof,
wherein the blocking agent is one or both of serine and dextran, and
wherein the pharmaceutical agent is a fibroblast growth factor.

15. A method for producing a bone filling material comprising:
a step for impregnating or pasting a blocking agent to a base material containing a calcium-based material,
a step for impregnating or pasting a pharmaceutical agent to the base material, the base material to which the blocking agent have been impregnated or pasted.

16. The method for producing a bone filling material according to claim 15,
wherein the step for impregnating or pasting a blocking agent is a step for impregnating or pasting the blocking agent to the base material containing a calcium-based material, the calcium-based material having been moisten and then dried, after sclerosing solution containing chondroitin sulfate have been impregnated or pastied to the calcium-based material.

17. The method for producing a bone filling material according to claim 15,
wherein the step for impregnating or pasting a pharmaceutical agent is a step for pasting a pharmaceutical agent to a certain site by the inkjet method.

18. The method for producing a bone filling material according to claim 15,
wherein the pharmaceutical agent is one or more kinds of osteogenesis/chondrongenesis promoter (including chondrongenesis promoting factor), joint disease therapeutic agent, bone/cartilage disease preventive/therapeutic agent, bone regenerating agent, osteoclastic inhibitor, angiogenetic promoter, antibacterial agent, antibiotics, and anticancer agent.

19. A drug release controlling carrier comprising:
a calcium-based material;
and a blocking agent of the calcium-based material.

20. The drug release controlling carrier according to claim 19,
wherein the calcium-based material is one or more kinds of hydroxyapatite, carbonate apatite, fluorapatite, chlorapatite, β-TCP, α-TCP, calcium metaphosphate, tetra-calcium phosphate, octa-calcium phosphate, calcium hydrogen phosphate, calcium dihydrogen phosphate, calcium pyrophosphate, salts thereof, and solvates thereof.

21. The drug release controlling carrier according to claim 19,
wherein the calcium-based material is: a compound of C₃-C₁₀ dicarboxylic acid and one or more kinds selecting from the group consisting of "hydroxyapatite, carbonate apatite, fluorapatite, chlorapatite, β-TCP, α-TCP, calcium metaphosphate, tetra-calcium phosphate, octa-calcium phosphate, calcium hydrogen phosphate, calcium dihydrogen phosphate, and calcium pyrophosphate"; a salt thereof; or a solvate thereof.

22. The drug release controlling carrier according to claim 19,
wherein the blocking agent includes one or more kinds of an amino acid, an amino acid derivative, or pharmaceutically acceptable salts thereof, the amino acid being selected from the group consisting of "alanine, arginine, asparagine, aspartic acid, cysteine, glutamine, glutamic acid, glycine, histidine, isoleucine, leucin, lysine, methionine, phenylalanine, proline, serine, threonine, tryptophan, tyrosin, and valine",
wherein a carboxyl group may be replaced with one of the groups represented by the formula (I) (in the formula (I) and hereinafter, R¹ represents C₁₋₆ alkyl group),
wherein an amino group may be replaced with -NHR¹, - N(R¹)₂, -SH or -R¹, and
wherein a hydroxyl group may be replaced with -NHCOR¹, -NHSO₂R¹, -NHCN, -CH₂ (CN), -CH (CN)₂, or -SH.
[formula (I)]

23. The drug release controlling carrier according to claim 19,
wherein the blocking agent includes one or more kinds of a saccharide, a saccharide derivative, or pharmaceutically acceptable salts thereof, the saccharide being selected from a group consisting of "pentose, hexose, heptose, octose, nonose, decose, maltose, cellobiose, gentiobiose, melibiose, lactose, turanose, sophorose, trehalose, isotrehalose, sucrose, isosaccharose, maltotriose, cellotriose, hyaluronic acid, teichoic acid, colominic acid, chondroitin sulfate, dermatan sulfate, heparin, heparan sulfate, keratan sulfate, chitin saccharide, pullulan, guar gum, lambda carrageenan, traganth gum, pectin, mannan, dextran, and maltodextran",
wherein a hydroxyl group may be replaced with -NHCOR¹, -NHSO₂R¹, -NHCN, -CH₂ (CN), -CH (CN)₂, or -SH (wherein R¹ represents C₁₋₆ alkyl group).

24. A medicament having controlled release of a pharmaceutical agent, the medicament comprising:
a drug release controlling carrier as claimed in claim 19; and
a pharmaceutical agent being impregnated or pasted to the drug release controlling carrier.
